(19) Europäisches Patentamt
European Patent Office
Office européen des brevets

(11) Numéro de publication : **0 466 585 A1**

# (12) DEMANDE DE BREVET EUROPEEN

(21) Numéro de dépôt : **91401915.3**

(22) Date de dépôt : **10.07.91**

(51) Int. Cl.[5] : **C07D 211/22,** C07D 211/14, C07D 211/70, C07D 211/12, C07D 207/20, C07D 401/04, C07D 405/04, C07D 207/08, A61K 31/445

(30) Priorité : **10.07.90 FR 9008729**

(43) Date de publication de la demande : **15.01.92 Bulletin 92/03**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Lavielle, Gilbert**
**1 avenue Lilly**
**F-78170 La Celle Saint Cloud (FR)**
Inventeur : **Laubie, Michel**
**35 avenue Foch**
**F-92420 Vaucresson (FR)**
Inventeur : **Colpaert, Francis**
**36 bis boulevard Carnot**
**F-78110 Le Vesinet (FR)**

## (54) Nouveaux dérivés de la pipéridine, de la tétrahydropyridine et de la pyrrolidine, leur procédé de préparation et les compositions pharmaceutiques qui les contiennent.

(57) L'invention concerne des composés de formule I :

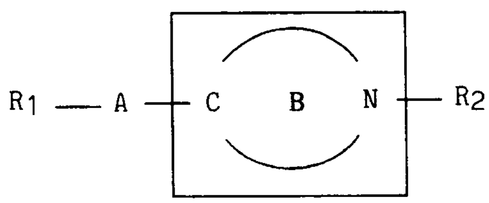

dans laquelle

- $R_1$ représente un radical naphtyle, un radical dihydronaphtyle, un radical tétrahydronaphtyle, un radical quinolyle ou un radical 1,4-benzodioxanyl,
- A représente une simple liaison, une double liaison, un radical méthylène, un radical de formule $z_1$ :

$$-CH= \qquad (z_1)$$

ou un radical de formule $z_2$ :

$$=CH- \qquad (z_2)$$

- le cycle B représente un radical pipéridyle, un radical pyrrolidinyle, ou un radical 1,2,3,6-tétrahydropyridyle

$R_2$ représente :

— un atome d'hydrogène, un radical benzyle, un radical alkyle de 1 à 6 atomes de carbone, à condition que dans l'un de ces cas $R_1$ soit différent d'un radical naphtyle ($y_1$), dihydronaphtyle ($y_2$) ou tétrahydronaphtyle ($y_3$) <u>et</u> B soit différent d'un radical pipéridinyl,

— un radical aminoalkyle de 1 à 6 atomes de carbone, un radical cyanoalkyle de 1 à 6 atomes de carbone ou un radical de formule $w_1$ :

EP 0 466 585 A1

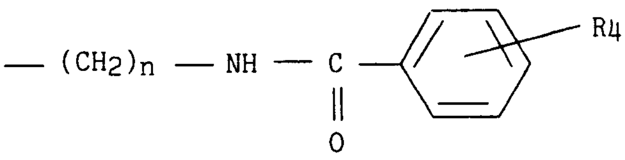

(w1)

(dans laquelle n est égal à 1-6 et $R_4$ représente un atome d'hydrogène, un atome d'halogène, un radical alkyle de 1 à 6 atomes de carbone ou un radical alcoxy de 1 à 6 atomes de carbone),
**et leurs sels d'addition** à un acide minéral ou organique pharmaceutiquement acceptable.
Médicaments.

La présente invention concerne des nouveaux dérivés de la pipéridine, de la tétrahydropyridine et de la pyrrolidine, leur procédé de préparation et les compositions pharmaceutiques qui les contiennent.

Certains dérivés de la 4-napht-1-yl 1,2,3,6-tétrahydropyridine ayant des propriétés antivirales sont décrits dans la littérature (Brevet EP 156,433).

Des dérivés de la 4-(3,4-dihydro-2-phényl-1-napht-1-yl) pipéridine ayant des propriétés contraceptives ou sédatives sont aussi connus (J. Med. Chem. (1967), 10(1), pp 78-84 ; Arzneim. Forsch., (1970), 20(9), pp 1235-1238 ; Int. J. Neuropharmacol. (1969), 8(2) pp 153-160) ; Acta. Pol. Pharm. (1967), Vol 24(5), pp 489-96 ; J-.Chem. Soc. C. (1969) 2 pp 217-22).

Des trifluorométhylphényltétrahydropyridines sont aussi utilisées pour la préparation de médicaments destinés à combattre les troubles anxio-depressifs.

Les composés de l'invention se distinguent des autres dérivés de la pipéridine, de la tétrahydropyridine et de la pyrrolidine décrits dans la littérature et mentionnés ci-dessus, par leurs structures originales et par leurs propriétés pharmacologiques.

Au niveau cardiovasculaire, les composés de l'invention diminuent la pression artérielle et la fréquence cardiaque. Cette action résulte d'une inhibition centrale du tonus sympathique et elle est reliée à leurs propriétés agonistes $5\text{-}HT_{1A}$.

Au niveau du système nerveux central, les composés de l'invention ont démontré des propriétés agonistes ou antagonistes $5\text{-}HT_{1A}$.

Ils peuvent donc être utiles dans le traitement de l'hypertension, de la migraine, de la dépression, de l'anxiété, de la schizophrénie, du stress et de la douleur.

La présente invention a plus particulierement pour objet les composés de formule générale I :

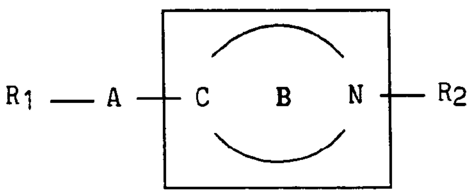

(I)

dans laquelle

- $R_1$ représente un radical naphtyle, de formule $y_1$ :

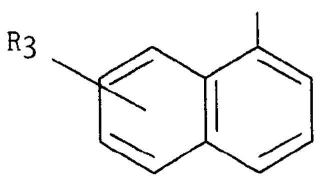

($y_1$)

un radical dihydronaphtyle, de formule $y_2$ :

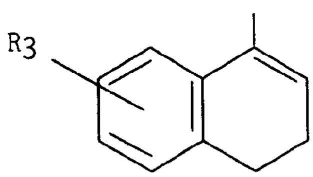

($y_2$)

un radical tétrahydronaphtyle de formule $y_3$ :

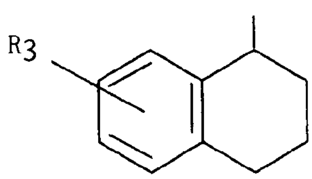

($y_3$)

(dans lesquelles $R_3$ représente un atome d'hydrogène, un atome d'halogène, un radical alkyle de 1 à 6 atomes de carbone, un radical hydroxy ou un radical alcoxy de 1 à 6 atomes de carbone), un radical quinol-3-yle (éventuellement substitué sur le cycle benzénique par un ou plusieurs atomes d'halogène, des radicaux alkyle de 1 à 6 atomes de carbone, des radicaux hydroxy, ou des radicaux alcoxy de 1 à 6 atomes de carbone), ou un radical 1,4-benzodioxan-5-yl,

- A représente une simple liaison, une double liaison, (à condition toutefois que $R_1$ représente un radical tétrahydronaphtyle), un radical méthylène, un radical de formule $z_1$ :

$$-CH= \qquad (z_1)$$

ou un radical de formule $z_2$ :

$$=CH- \qquad (z_2)$$

(à condition toutefois que dans ce cas, $R_1$ représente un radical tétrahydronaphtyle),

- le cycle B représente un radical pipéridyle (à condition toutefois que dans ce cas A représente une simple ou une double liaison), un radical pyrrolidinyle (à condition toutefois que dans ce cas A représente un radical méthylène, un radical $z_1$ ou un radical $z_2$), ou un radical 1,2,3,6-tétrahydropyridyle (à condition toutefois que dans ce cas A représente une simple liaison),

$R_2$ représente :

– un atome d'hydrogène, un radical benzyle, un radical alkyle de 1 à 6 atomes de carbone, à condition que dans l'un de ces cas $R_1$ soit différent d'un radical naphtyle ($y_1$), dihydronaphtyle ($y_2$) ou tétrahydronaphtyle ($y_3$) et B soit différent d'un radical pipéridinyl,

– un radical aminoalkyle de 1 à 6 atomes de carbone, un radical cyanoalkyle de 1 à 6 atomes de carbone ou un radical de formule $w_1$ :

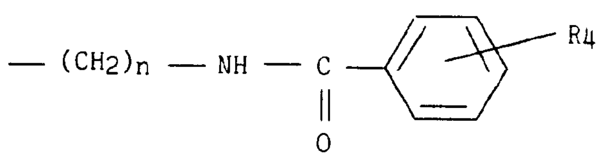

($w_1$)

(dans laquelle :

n est égal à 1-6

et

$R_4$ représente un atome d'hydrogène, un atome d'halogène, un radical alkyle de 1 à 6 atomes de carbone ou un radical alcoxy de 1 à 6 atomes de carbone),

à condition toutefois que

– quand $R_1$ est un radical $y_2$, $R_3$ représente un atome d'hydrogène, A une simple liaison et B un radical pipéridyle, $R_2$ ne représente pas simultanément un radical méthyle,

et

– quand $R_2$ est un radical $y_1$, $R_3$ représente un atome d'hydrogène, A une simple liaison et B un radical 1,2,3,6-tétrahydropyridyle, $R_2$ ne représente pas simultanément un atome d'hydrogène,

et

– quand $R_1$ est un radical $y_3$, $R_3$ représente un atome d'hydrogène, A une double liaison et B un radical pipéridyle, $R_2$ ne représente pas simultanément un radical méthyle,

**leurs stéréoisomères possibles,**

**et leurs sels d'addition** à un acide minéral ou organique pharmaceutiquement acceptable.

La présente invention a également pour objet un procédé de préparation des composés de formule générale I, caractérisé en ce que :

soit

l'on fait réagir un composé de formule II :

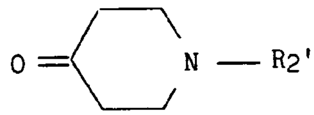

(II)

dans laquelle $R_{2'}$ représente un radical alkyle de 1 à 6 atomes de carbone, ou un radical benzyle, avec un composé de formule III :

$R_1—X$ (III)

dans laquelle $R_1$ a la même signification que pour la formule I et X représente un atome de brome, de lithium ou un groupement MgBr, pour former les composés de formule IV :

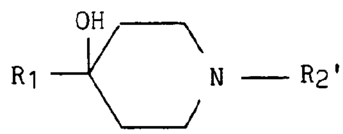

(IV)

dans laquelle $R_1$ a la signification indiquée ci-dessus et $R_{2'}$ représente un radical alkyle de 1 à 6 atomes de carbone ou un radical benzyle,

que l'on soumet à l'action de l'acide bromhydrique pour former les composés de formule $I_a$ :

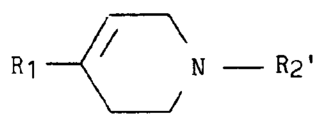

($I_a$)

dans laquelle $R_1$ a la même signification que pour la formule I et $R_{2'}$ représente un radical alkyle de 1 à 6 atomes de carbone ou un radical benzyle,

et ensuite

- soit

les composés de formule $I_a$ sont soumis à l'action du chloroformiate d'éthyle en présence d'un sel minéral alcalin pour former les composés de formule V :

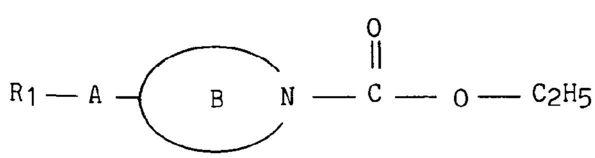

(V)

dans laquelle $R_1$ a la même signification que pour la formule I, A est une simple liaison et B représente un radical 1,2,3,6-tétrahydropyridyle,

- soit

les composés de formule $I_a$ sont d'abord soumis à une hydrogénation catalytique pour former les composés de formule $I_b$ :

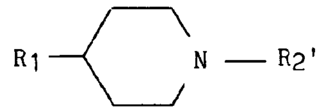

$(I_b)$

dans laquelle $R_1$ a la même signification que pour la formule I, et $R_{2'}$ représente un radical alkyle de 1 à 6 atomes de carbone ou un radical benzyle,

puis, à l'action du chloroformiate d'éthyle en présence d'un sel minéral alcalin pour former les composés de formule V,

dans laquelle $R_1$ a la même signification que pour la formule I et B représente un radical pipéridyle,

soit

l'on fait réagir un composé de formule VI :

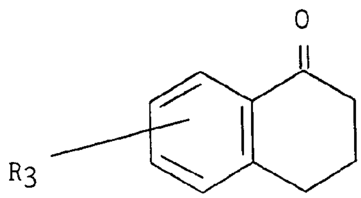

(VI)

dans laquelle $R_3$ a la même signification que pour la formule I,

ou

avec un composé de formule VII,

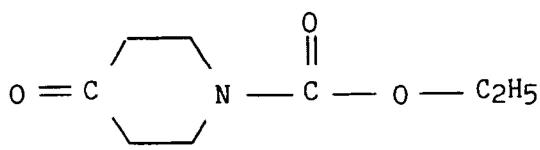

(VII)

pour former les composés de formule V dans laquelle $R_1$ représente un radical tétrahydronaphtyle de formule $y_3$, A est une double liaison et B représente un radical pipéridyle,

ou

avec un composé de formule VIII :

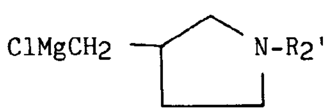

(VIII)

dans laquelle $R_{2'}$ a la même signification que pour la formule II,
pour former les composés de formule IX :

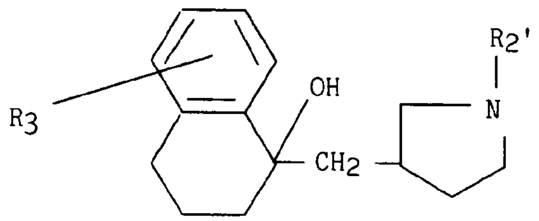

(IX)

dans laquelle la signification de $R_3$ et $R_{2'}$ reste identique à celle donnée ci-dessus,

que l'on soumet à l'action de l'acide para-toluènesulfonique pour former les composés de formule $I_c$ et $I_d$ :

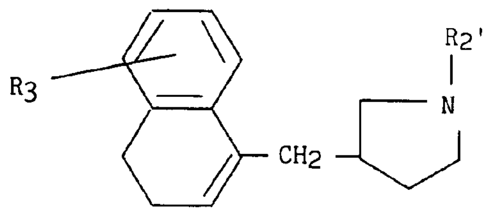

($I_c$)

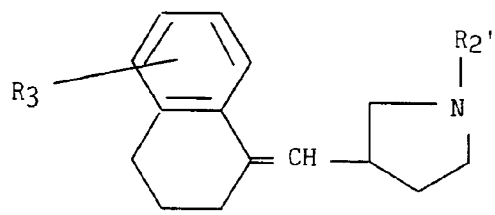

(Id)

dans lesquelles $R_3$ a la même signification que pour la formule I et la signification de $R_{2'}$ est identique à celle donnée pour la formule II,

lesquels ensuite sont séparés

puis soumis à l'action du chloroformiate d'éthyle en présence d'un sel minéral alcalin pour former les composés de formule V, dans laquelle $R_1$ représente un radical dihydronaphtyle de formule $y_2$ ou un radical tétrahydronaphtyle de formule $y_3$, A est respectivement un radical méthylène ou un radical de formule $z_2$ et B représente un radical pyrrolidinyle,

ou

avec un composé de formule X :

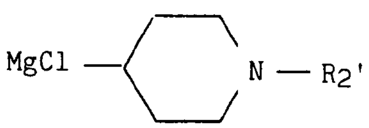

(X)

dans laquelle $R_{2'}$ a la même signification que pour la formule II,

pour former les composés de formule XI :

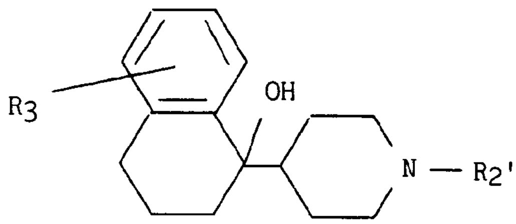

(XI)

dans laquelle la signification de $R_3$ reste identique à celle donnée pour la formule I et $R_{2'}$ représente un radical benzyle ou un radical alkyle de 1 à 6 atomes de carbone,

lesquels ensuite sont soumis à l'action de l'acide paratoluènesulfonique pour former les composés de formule $I_e$ :

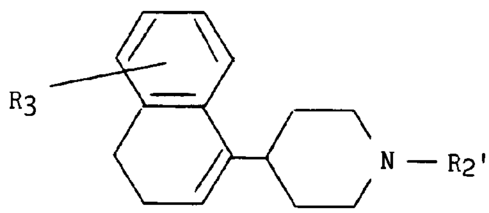

($I_e$)

dans laquelle $R_3$ a la même signification que pour la formule I et $R_{2'}$ représente un radical alkyle de 1 à 6 atomes de carbone ou un radical benzyle,

lesquels ensuite

- soit

l'on fait réagir avec le chloroformiate d'éthyle pour former les composés de formule V dans laquelle $R_1$ représente un radical dihyronaphtyle de formule $y_2$, A est une simple liaison et B représente un radical pipéridyle,

- soit

l'on soumet à l'action de tétrachloro 1,2-benzoquinone, pour former les composés de formule $I_f$ :

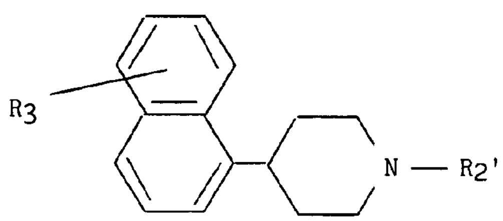

($I_f$)

dans laquelle $R_3$ a la même signification que pour la formule I et $R_{2'}$ représente un radical alkyle de 1 à 6 atomes de carbone ou un radical benzyle,

lesquels ensuite

l'on fait réagir avec le chloroformiate d'éthyle pour former les composés de formule V dans laquelle $R_1$ représente un radical naphtyle de formule $y_1$, A est une simple liaison et B représente un radical pipéridyle,

et ensuite

l'on soumet les composés de formule V, soit à l'action de l'acide bromhydrique, soit, à l'action de l'hydroxyde de potassium, pour former les composés de formule $I_g$ :

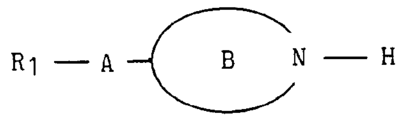

$(I_g)$

dans laquelle la signification de $R_1$, A et B reste identique à celle donnée pour la formule I, lesquels ensuite l'on fait réagir avec un composé de formule XII :

$$Br\text{-}(CH_2)_{n-1}\text{-}CN \qquad (XII)$$

dans laquelle n a la même signification que pour la formule I, pour former les composés de formule $I_h$ :

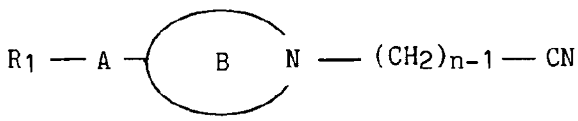

$(I_h)$

dans laquelle $R_1$, A, B et n ont la même signification que pour la formule I,

lesquels ensuite sont soumis à l'action d'hydrure de lithium et d'aluminium pour former les composés de formule $I_i$ :

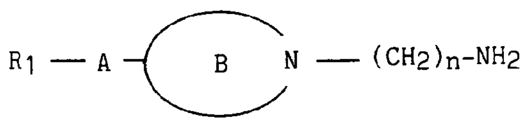

$(I_i)$

dans laquelle la signification de $R_1$, A, B et n reste identique à celle donnée pour la formule I, que l'on fait réagir avec un composé de formule XIII

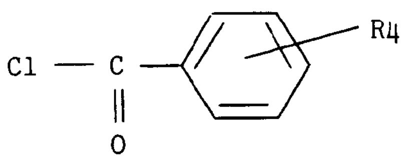

(XIII)

dans laquelle la signification de $R_4$ reste identique à celle donnée pour la formule I, pour former les composés de formule $I_j$ :

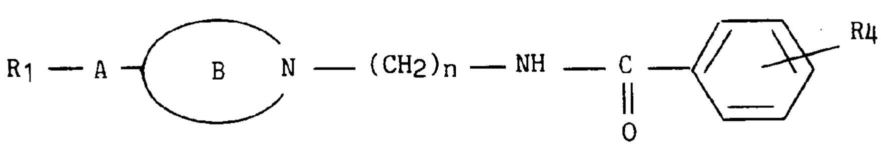

$(I_j)$

dans laquelle $R_1$, A, B, n et $R_4$ ont la même signification que pour la formule I,

et ensuite

l'on sépare les composés de formule $I_a$-$I_j$ (qui font l'ensemble des composés de formule I) en leurs stéréoisomères possibles,

et/ou l'on salifie avec un acide organique ou minéral, pharmaceutiquement acceptable, pour former les sels d'addition correspondants.

La réaction des composés de formule II avec les composés de formule III est réalisée en présence de butyllithium, dans le tétrahydrofurane ou un autre solvant chimiquement équivalent, et à une température comprise entre - 60°C et -78°C.

De manière préférentielle, on utilise comme catalyseur, lors de l'hydrogénation des composés de formule $I_a$, pour former les composés de formule $I_b$, du palladium sur charbon.

Lors de la réaction des composés de formule $I_a$, $I_b$, $I_c$, $I_d$, $I_e$ et $I_f$ avec le chloroformiate d'éthyle, on utilise comme sel minéral alcalin, l'hydrogénocarbonate de sodium.

La réaction des composés de formule VI avec les composés de formule VII est réalisée en présence du complexe trichlorure de titane/diméthoxyéthane et du couple zinc-cuivre (préparé selon le procédé décrit dans J.Org.Chem. (1989), 54, p. 3749).

La réaction des composés de formule IX et XI avec l'acide paratoluènesulfonique est réalisée à chaud, dans le dichlorométhane ou dans un autre solvant aliphatique halogéné de faible poids moléculaire ($C_1$-$C_3$).

Pour former les composés de formule $I_f$, l'oxydation des composés de formule $I_e$ est réalisée à l'aide de la tétrachloro 1,2-benzoquinone, à chaud, dans un solvant alcoolique anhydre.

Pour former les composés de formule $I_g$, les composés de formule V sont soumis à chaud soit à l'action d'hydroxyde de potassium en solution dans l'éthanol, soit à l'action de l'acide bromhydrique à 48 %.

La réaction des composés de formule $I_i$ avec les composés de formule XIII est réalisée à température ambiante.

Parmi les acides pharmaceutiquement acceptables pour la préparation des sels d'addition aux composés de formule générale I, on peut citer les acides chlorhydrique, phosphorique, fumarique, citrique, oxalique, sulfurique, ascorbique, tartrique, maléïque, mandélique, méthane-sulfonique, etc....

Les composés de la présente invention possèdent des propriétés pharmacologiques fort intéressantes. Au niveau cardiovasculaire, les composés de l'invention diminuent la pression artérielle et la fréquence cardiaque chez le rat et chez le chien. Cette action résulte d'une inhibition centrale du tonus sympathique. En effet, les essais pharmacologiques ont démontré que la baisse de pression provoquée par l'administration i.v. des composés de l'invention chez le chien, s'accompagne d'une forte diminution de l'activité électrique du nerf sympathique rénal.

Cette diminution centrale du tonus sympathique résulte d'une activation des récepteurs $5HT_{1A}$ centraux au niveau du noyau rétrofacial (Eur.Journal of Pharm.,(1989),160,p.385-294). Les essais pharmacologiques ont aussi prouvé que les composés de l'invention sont plus actifs que le flesinoxan, composé de référence ayant des propriétés antihypertensives, dues à son activité agoniste des récepteurs $5\text{-}HT_{1A}$ (European Journal of Pharmacology,(1988),149,p.213-223). D'autre part, les composés de l'invention ont une activité bénéfique au niveau rénal (T.I.P.S.,(1989),10,p.469-471).

Les essais de binding ont confirmé que les composés de l'invention se comportent aussi comme de très puissants ligands des récepteurs $5\text{-}HT_{1A}$, avec une activité, agoniste ou antagoniste au niveau système nerveux central.

Les composés de l'invention trouvent donc leur application dans le traitement du stress (Neuropharmac.,(1989),Vol.25,N°5,p.471-476), de la migraine (T.I.P.S.,(1989),Vol.10,pp.200-204), de l'anxiété, de la dépression, de la schizophrénie et de la douleur (Pharmacology and Toxicology,(1989),64,p.3-5), (Drugs of the Future,(1988),13,N°5, p.429-437), (J.Neural.Transm.,(1988),74,p.195-198).

Les composés actifs au niveau des récepteurs $5\text{-}HT_{1A}$ peuvent aussi modifier le comportement alimentaire et sexuel (Jour. of Receptor Research, (1988),8,p.59-81).

L'invention s'étend aussi aux compositions pharmaceutiques renfermant comme principe actif au moins un composé de formule générale I, ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut varier largement en fonction de l'âge, du poids du patient, de la nature et de la sévérité

de l'affection ainsi que de la voie d'administration. D'une manière générale, la posologie unitaire s'échelonnera entre 0,1 et 100 mg et la posologie journalière, utilisable en thérapeutique humaine, entre 0,1 et 500 mg. La voie d'administration préférée est la voie orale ou parentérale.

Les exemples suivants, donnés à titre non-limitatif, illustrent l'invention.

Les points de fusion ont été mesurés selon la technique Micro-Köfler.

Les spectres de résonance magnétique nucléaire du proton (RMN-$^1$H) des composés de formule générale I ont été enregistrés selon le cas à 200 et 400 mHz et sont indiqués dans le Tableau I.

**EXEMPLE 1**

**Bromhydrate de la 1-méthyl 4-(napht-1-yl) 1,2,3,6-tétrahydropyridine**

**Stade A**

4-Hydroxy 1-méthyl 4-(napht-1-yl) pipéridine

Une solution de 51,75 g de 1-bromonaphtalène dans 250 ml de tétrahydrofurane est additionnée sous flux d'azote goutte à goutte et à -78°C, à une solution de 175 ml de butyllithium (1,6 M dans l'hexane) dans 500 ml de tétrahydrofurane.

Après 1 heure d'agitation, une solution de 28,29 g de 1-méthyl pipérid-4-one dans 250 ml de tétrahydrofurane est ajoutée goutte à goutte.

Le milieu réactionnel est agité 1 heure à -78°C, puis 5 heures à 20°C, et ensuite hydrolysé avec 100 ml d'une solution saturée de chlorure d'ammonium.

L'extraction au dichlorométhane suivie de l'évaporation du solvant, fournit un résidu huileux qui, repris dans l'éther éthylique cristallise sous forme d'un solide blanc.

– Rendement : 73 %
– Point de fusion : 160°C

Spectre de résonance magnétique nucléaire du proton (solvant $CDCl_3$) : 2,2 ppm **s+m** 3H+4H ; 2,65 ppm **m** 4H ; 7,35 ppm **m** 4H ; 7,75 ppm **d** 1H ; 7,85 ppm **m** 1H ; 8,9 ppm **m** 1H.

**Stade B**

49 g du composé obtenu au stade précédent en suspension dans 1 litre d'une solution d'acide bromhydrique à 48 % sont portés au reflux 24 heures. Le résidu obtenu après évaporation est repris 3 fois avec de l'éthanol puis avec de l'acétone pour fournir après filtration et séchage 47,94 g de bromhyrate de la 1-méthyl 4-napht-1-yl 1,2,3,6-tétrahydropyridine.

– Rendement : 86,65 %
– Point de fusion : > 260°C

**EXEMPLE 2**

**Bromhydrate de la 1-méthyl 4-napht-1-yl pipéridine**

20 g du composé de l'exemple 1 sont réduits par 1680 ml d'hydrogène sous pression atmosphérique et température ambiante, en présence de 1 g de palladium sur charbon à 10 %, dans un litre d'éthanol et 100 ml de diméthylformamide.

Après filtration du catalyseur et évaporation des solvants, on obtient un produit brut, qui repris dans l'acétone, fournit 19 g de bromhydrate attendu.

– Rendement : 95,5 %
– Point de fusion : > 260°C

**EXEMPLE 3**

**Bromhydrate de la 4-napht-1-yl pipéridine**

**Stade A**

[4-(napht-1-yl) pipérid-1-yl] carbamate d'éthyle

19 g du sel obtenu dans l'exemple 2 sont transformés en 1-méthyl 4-napht-1-yl pipéridine à l'aide de

l'hydroxyde de sodium dans le dichlorométhane.

La base ainsi obtenue est immédiatement mise en présence de 10 g d'hydrogénocarbonate de sodium dans 500 ml de toluène, sous flux d'azote. 100 ml de chloroformiate d'éthyle sont ajoutés par fractions au milieu réactionnel, puis le tout est porté au reflux pendant 24 heures. La filtration suivie de l'évaporation conduit à 10,7 g d'une huile.

– Rendement : 61,14 %

**Stade B**

10,5 g du composé obtenu au stade A dans 300 ml d'une solution d'acide bromhydrique à 48 % sont portés au reflux pendant 20 heures. Après évaporation et 2 lavages avec de l'éthanol, le résidu est repris avec de l'acétone puis filtré pour donner le bromhydrate de la 4-(napht-1-yl) pipéridine.

– Rendement : 86,64 %
– Point de fusion : > 260°C

**EXEMPLE 4**

**Chlorhydrate de (4-napht-1-yl pipérid-1-yl) acétonitrile**

5 g du composé de l'exemple 3, 7,088 g de carbonate de potassium et 2,259 g de bromoacétonitrile dans 200 ml d'acétone, sont agités pendant 10 heures à reflux sous atmosphère d'azote. Après filtration et évaporation du filtrat, le résidu obtenu est traité par une solution d'éthanol chlorhydrique pour donner 4,5 g du chlorhydrate attendu.

– Rendement : 91,83 %
– Point de fusion : 172°C

**EXEMPLE 5**

**Chlorhydrate de la 1-[2-(4-fluorobenzamido) éth-1-yl] 4-napht-1-yl pipéridine**

**Stade A**

1-(2-aminoéth-1-yl) 4-napht-1-yl pipéridine

1,75 g d'hydrure de lithium et d'aluminium sont ajoutés par fractions sous flux d'azote à une solution de 4,4 g du composé de l'exemple 4 dans 150 ml de tétrahydrofurane. Après 1 heure d'agitation, le milieu est hydrolysé avec 1,75 ml d'eau, 3,5 ml d'une solution d'hydroxyde de sodium à 10 % (p/p) et 7 ml d'eau. La filtration sur célite, suivie de l'évaporation du solvant, fournit un produit huileux utilisé tel quel dans le stade suivant.

– Rendement : quantitatif

**Stade B**

Une solution de 1,81 g de triéthylamine dans 40 ml de tétrahydrofurane est ajoutée goutte à goutte à -5°C, sous flux d'azote, à une solution de 3,8 g du composé obtenu au stade précédent dans 180 ml de tétrahydrofurane. Dans les mêmes conditions, une solution de 2,84 g de chlorure de 4-fluoro benzoyle dans 40 ml de tétrahydrofurane est additionnée au milieu réactionnel. Après avoir agité 1 heure à température ambiante, on hydrolyse avec une solution saturée d'hydrogénocarbonate de sodium. La décantation suivie du séchage de la phase organique sur sulfate de sodium et de l'évaporation du solvant, conduit à un produit qui, repris dans l'éther éthylique cristallise sous forme d'un solide blanc.

3,3 g du chlorhydrate correspondant sont obtenus après addition d'une solution d'éthanol chlorhydrique.

– Rendement : 64 %
– Point de fusion : 260°C

**EXEMPLE 6**

**Chlorhydrate de la 1-[2-(4-fluorobenzamido) éth-1-yl] 4-napht-1-yl 1,2,3,6-tétrahydropyridine.**

**Stade A**

(4-napht-1-yl 1,2,3,6-tétrahydro pyrid-1-yl) carbamate d'éthyle

22,3 g du composé de l'exemple 1 sont traités par 50 ml de chloroformiate d'éthyle et 30 g d'hydrogénocarbonante de sodium, selon le procédé décrit dans l'exemple 3 stade A pour donner le produit attendu.

– Rendement : 53,88 % Spectre de résonance magnétique nucléaire du proton (solvant $CDCl_3$) : 1,3 ppm **t** 3H ; 2,55 ppm **m** 2H ; 3,8 ppm **m** 2H ; 4,1-4,3 ppm **q+m+m** 2H+1H+1H ; 5,8 ppm **m** 1H ; 7,25 ppm **dd** 1H ; 7,35-7,55 ppm **m** 3H ; 7,7-8 ppm **m+d** 2H+1H.

**Stade B**

Bromhydrate de la 4-napht-1-yl 1,2,3,6-tétrahydropyridine

Le traitement de 11 g du carbamate obtenu au stade précédent par une solution d'acide bromhydrique à 48 % fournit 9,02 g du bromhydrate de la 4-napht-1-yl 1,2,3,6-tétrahydropyridine.

– Rendement : 80 %

– Point de fusion : > 260°C

**Stade C**

Chlorhydrate de (4-napht-1-yl 1,2,3,6-tétrahydropyrid-1-yl) acétonitrile

Ce sel a été obtenu à partir du composé obtenu au stade B en utilisant le procédé décrit dans l'exemple 4.

– Rendement : 80 %

**Stade D**

1-(2-aminoéth-1-yl) 4-napht-1-yl 1,2,3,6-tétrahydropyridine

6,25 g du chlorhydrate préparé précédemment sont réduits par 2,5 g d'hydrure de lithium et d'aluminium et fournissent le composé aminé attendu, qui est utilisé immédiatement dans le stade suivant.

– Rendement : quantitatif

**Stade E**

Le traitement de 5,5 g d'amine préparée au stade précédent, par 2,66 g de triéthylamine et 4,18 g de chlorure de 4-fluorobenzoyle conduit à un produit brut qui est purifié par chromatographie sur colonne de gel de silice (70-230 mesh) en utilisant comme solvant d'élution un mélange de dichlorométhane, de méthanol et d'amoniaque 97 : 3 : 0,3 (V/V/V).

Le produit ainsi obtenu est transformé en chlorhydrate.

– Rendement : 37 %

– Point de fusion : 233°C

**EXEMPLE 7**

**Dibromhydrate de la 3-(1-méthyl 1,2,3,6-tétrahydropyrid-4-yl) quinoléine**

**Stade A**

3-(4-hydroxy 1-méthyl pipérid-4-yl) quinoléine

95,01 g de 3-bromoquinoléine ont été traitées par 200 ml d'une solution à 2,5 m de butyllithium dans l'hexane et 51,62 g de 1-méthyl pipérid-4-one selon le procédé décrit dans l'exemple 1 stade A, pour obtenir le produit attendu.

– Rendement : 53,84 %

– Point de fusion : 175°C

Spectre de résonance magnétique nucléaire du proton (solvant $CDC1_3$) : 2,3 ppm **t+d** 2H ; 2,4 ppm **s** 3H ; 2,55

ppm t+d 2H ; 2,85 ppm d 2H ; 7,7 et 7,55 ppm m+m 2H ; 7,8 ppm d 1H ; 8,10 ppm d 1H ; 8,25 ppm d 1H ; 9,1 ppm d 1H.

**Stade B**

Le dibromhydrate de la 3-(1-méthyl 1,2,3,6-tétrahydropyrid-4-yl) quinoléine a été obtenu en traitant 53 g du composé obtenu au stade A par une solution d'acide bromhydrique à 48 %.

– Rendement : 72,80 %
– Point de fusion : > 260°C

**EXEMPLE 8**

**Chlorhydrate de la 3-(1,2,3,6-tétrahydropyrid-4-yl) quinoléine**

Ce produit a été préparé à partir du composé de l'exemple 7 et en utilisant le procédé décrit dans l'exemple 6, stades A et B. Le composé obtenu a été salifié par l'alcool chlorhydrique.

– Rendement : 65 %
– Point de fusion : > 260°C

**EXEMPLE 9**

**Dibromhydrate de la 3-(1-méthyl pipérid-4-yl) quinoléine.**

L'hydrogénation du composé de l'exemple 7, selon le procédé décrit dans l'exemple 2, fournit le composé attendu.

– Rendement : 95,4 %
– Point de fusion : > 260°C

**EXEMPLE 10**

**Dibromhydrate de la 3-pipérid-4-yl quinoléine**

Ce produit a été préparé à partir du composé de l'exemple 9 en utilisant le procédé décrit dans l'exemple 3.

– Rendement : 74 %
– Point de fusion : > 260°C

**EXEMPLE 11**

**Dichlorhydrate de [4-(quinol-3-yl) pipérid-1-yl] acétonitrile**

Le traitement de 10,90 g du composé de l'exemple 10 par 2,3 ml de bromoacétonitrile et 16 g de carbonate de potassium fournit 9,08 g de produit attendu, sous forme de base.
Pour la salification on utilise l'éthanol chlorhydrique.

– Rendement : 85,77 %
– Point de fusion : 210 - 214°C

**EXEMPLE 12**

**Dichlorhydrate de la 1-[2-(4-fluorobenzamido) éth-1-yl] 4-quinol-3-yl pipéridine**

Ce produit a été préparé à partir du composé de l'exemple 11 et selon le procédé décrit dans l'exemple 5.

– Rendement : 50 %
– Point de fusion : 229°C

**EXEMPLE 13**

**1-benzyl 3-[3,4-dihydro 7-méthoxy napht-1-yl) méthylènyl] pyrrolidine**

**Stade A**

1-benzyl 3-[(1-hydroxy 7-méthoxy 1,2,3,4-tétrahydro napht-1-yl), métylènyl] pyrrolidine

Ajouter sous azote 40 g de 3-chlorométhyl-1-benzyl pyrrolidine à une suspension de 4,63 g de magnésium dans 100 ml d'éther éthylique anhydre.

Diluer peu à peu le milieu réactionnel avec 300 ml de benzène au fur et à mesure que le magnésium disparait. Porter 1 heure à reflux, puis refroidir à 0°C et additionner une solution de 37 g de 7-méthoxy- 1,2,3,4-tétrahydro napht-1-one dans 120 ml de benzène.

Laisser remonter la température à 20°C, hydrolyser après 36 heures, avec 200 ml d'une solution saturée de chlorure d'ammonium.

Après séchage et concentration de la phase organique, l'huile obtenue est purifiée par chromatographie sur silice en utilisant comme éluant, un mélange de dichlorométhane et de méthanol 100 : 3 (V/V).

– Rendement : 48 %

Spectre de résonance magnétique nucléaire du proton (solvant $CDCl_3$) : 1,65 à 2,1 ppm **m+m+m+d+dd** 1H+2H+2H+2H+1H ; 2,15 à 2,5 ppm **m+m+m** 1H+1H+1H
2,6 à 2,85 ppm **m+m+dd** 2H+1H+1H ; 3,55 ppm **dd** 2H ; 3,75 ppm **s** 3H ; 6,7 ppm **dd** 1H ; 7,0 ppm **d** 1H ; 7,05 ppm **d** 1H ; 7,1 à 7,4 ppm **m** 5H.

**Stade B**

Porter à reflux 30 minutes, une solution de 20 g de produit obtenu au stade A et 11,9 g d'acide para-toluènesulfonique monohydraté, dans 600 ml de dichlorométhane.

Relarguer ensuite l'amine avec une solution d'hydroxyde de sodium et concentrer la phase organique.

L'huile obtenue est purifiée par chromatographie sur silice (70-230 **M**esh) en utilisant comme éluant, un mélange de méthanol et de dichlorométhane 3 : 100 (V/V).

12 g de 1-benzyl 3-[3,4-dihydro 7-méthoxy napht-1-yl) méthylènyl] pyrrolidine sont ainsi obtenus.

**EXEMPLE 14**

**1-benzyl 3-[(7-méthoxy 1,2,3,4-tétrahydronapht-1-yl) méthylidyn] pyrrolidine**

3 g de ce composé ont été obtenus lors de la purification par chromatographie de l'huile obtenue au stade B de l'exemple 13.

**EXEMPLE 15**

**4-(3,4-dihydro 7-méthoxy napht-1-yl) 1-méthyl pipéridine**

**Stade A**

4-(1-hydroxy 7-méthoxy 1,2,3,4-tétrahydro napht-1-yl) 1-méthyl pipéridine

Une solution de 40 g de 4-chloro 1-méthylpipéridine dans 150 ml de tétrahydrofurane est ajoutée à une suspension de 6,9 g de magnésium dans 50 ml de tétrahydrofurane.

La formation du magnésien est complétée en portant 2 heures à reflux.

Une solution de 32,2 g de 7-méthoxy 1,2,3,4-tétrahydro napht-1-one dans 150 ml de tétrahydrofurane est ensuite ajoutée à 10°C.

Le milieu réactionnel est maintenu sous agitation, à température ambiante pendant 15 heures, puis hydrolysé à 0°C par 15 g de chlorure d'ammonium en solution dans 80 ml d'eau.

Après évaporation du solvant sous vide, le résidu est repris dans l'eau, acidifié, lavé à l'éther éthylique. La phase aqueuse est ensuite basifiée et le produit extrait par 400 ml de toluène est lavé par 200 ml d'eau.

Après concentration sous vide, l'huile obtenue cristallise.

– Point de fusion : > 60°C

**Stade B**

Une solution de 15 g de l'huile obtenue au stade A dans 400 ml de dichlorométhane anhydre est portée à reflux 20 minutes en présence de 11,4 g d'acide para-toluènesulfonique.

Après refroidissement le milieu réactionnel est lavé par 20 ml d'une solution d'hydrogénocarbonate de sodium saturée. La phase organique est séchée sur sulfate de sodium puis concentrée sous vide. On obtient une huile.

– Rendement : 40 %

**EXEMPLE 16**

**4-(7-méthoxy napht-1-yl) 1-méthyl pipéridine**

Un mélange du 11,5 g de produit de l'exemple 15 et de 23,1 g de tétrachloro 1,2-benzoquinone en solution dans 600 ml d'alcool éthylique anhydre est porté à reflux pendant 30 heures.

Après concentration sous vide, le résidu est repris dans l'eau puis extrait au dichlorométhane, séché sur sulfate de sodium anhydre et concentré sous vide.

Le produit obtenu est purifié sur colonne d'alumine neutre à l'aide d'un mélange de dichlorométhane et de méthanol 99,5 : 0,5 (V/V).

– Rendement : 82 %

**EXEMPLE 17**

**Chlorhydrate de 4-(7-méthoxy napht-1-yl) pipéridine**

**Stade A**

[4-(7-méthoxy napht-1-yl) pipérid-1-yl] carbamate d'éthyle

Le composé de l'exemple 16 est mis en solution dans 200 ml de xylène anhydre en présence de 3,9 g de carbonate de sodium, et 17,6 ml de chloroformiate d'éthyle sont additionnés à 20°C. Le mélange est porté à reflux pendant 24 heures.

Le milieu réactionnel est refroidi puis hydrolysé par une solution d'acide chlorhydrique 2N.

La phase organique est lavée par une solution de soude diluée, séchée sur sulfate de sodium et concentrée sous vide.

– Rendement : 88 %

**Stade B**

Un mélange de 8,9 g de carbamate obtenu au stade A, 12,7 g d'hydroxyde de potassium, 30 ml d'eau et 200 ml d'alcool éthylique est porté à reflux pendant 40 heures.

Après évaporation sous vide, le résidu est repris dans l'eau et extrait au dichlorométhane.

La phase organique est séchée sur sulfate de sodium et concentrée sous vide.

L'huile obtenue est salifiée par 3,12 ml d'éthanol chlorhydrique 7,6 N.

– Rendement : 55 %

– Point de fusion : 240°C

**EXEMPLE 18**

**Chlorhydrate de 1-[2-(4-fluorobenzamido) éth-1-yl] 4-(7-méthoxy napht-1-, yl) pipéridine**

Ce produit a été préparé à partir du composé de l'exemple 17 et en utilisant successivement les procédés décrits dans les exemples 4 et 5.

– Rendement : 25 %

– Point de fusion : 215°C

**EXEMPLE 19**

**4-(7-méthoxy 1,2,3,4-tétrahydronapht-1-yl) pipérid-4-ylidène**

**Stade A**

[4-(7-méthoxy 1,2,3,4-tétrahydronapht-1-yl) pipérid-4-ylidène] carbamate d'éthyle

Successivement, 921 g de complexe trichlorure de titane-diméthoxyéthane 1 M : 1,5 M puis, 888 g du couple zinc/cuivre (préparé selon le procédé décrit par J.Mc.Murry. J. Org. Chem. (1989), 54, p. 3749), sont versés, sous flux d'argon, sur 8 litres de diméthoxyéthane. Le mélange est porté à reflux pendant 13 heures puis refroidi à 20°C.

Une solution composée de 31 g de 7-méthoxy 1,2,3,4-tétrahydro napht-1-one, de 60,3 g de (4-oxo pipérid-1-yl) carbamate d'éthyle et de 400 ml de diméthoxyéthane, est ajoutée, goutte à goutte en 5 heures dans le milieu réactionnel. Celui-ci est ensuite porté à nouveau à reflux pendant 15 heures, puis refroidi et dilué avec 3 litres de dichlorométhane.

Après filtration sur célite, puis concentration sous vide, le résidu obtenu est repris dans 800 ml de dichlorométhane.

L'insoluble est éliminé par filtration et la phase organique évaporée sous vide.

L'huile obtenue est purifiée par chromatographie sur colonne de silice en utilisant comme éluant le dichlorométhane.

– Rendement : 55 %

Spectre de résonance magnétique nucléaire du proton (solvant $CDCl_3$) : 1,25 ppm **t** 3H ; 1,75 ppm **q** 2H ; 2,45 ppm **m** 4H ; 2,6 ppm **t** 2H ; 2,65 ppm **t** 2H ; 3,4 ppm **t** 2H ; 3,55 ppm **t** 2H ; 3,8 ppm **s** 3H ; 4,2 ppm **q** 2H ; 6,7 ppm **m** 2H ; 7,0 ppm **d** 1H.

**Stade B**

2,6 g du composé obtenu au stade A ont été traités selon le procédé décrit dans l'exemple 17 stade B pour obtenir le sel attendu

– Rendement : 63 %
– Point de fusion : > 260°C

**EXEMPLE 20**

**[4-(7-méthoxy 1,2,3,4-tétrahydronapht-1-yl) pipérid-4-ylidène] acétonitrile**

Ce composé a été préparé à partir du composé de l'exemple 19 et en utilisant le procédé décrit dans l'exemple 4.

– Rendement : 94 %

**EXEMPLE 21**

**Chlorhydrate de 1-[2-(4-fluorobenzamido) éth-1-yl] 4-(7-méthoxy 1,2,3,4-tétrahydro napht-1-yl) pipérid-4-ylidène**

Ce composé a été préparé à partir du composé de l'exemple 20 et en utilisant le procédé décrit dans l'exemple 5.

– Rendement : 80 %
– Point de fusion : 120°C

**EXEMPLE 22**

**1-benzyl 4-(1,4-benzodioxan-5-yl) 1,2,3,4-tétrahydropyridine**

**Stade A**

1-benzyl 4-hydroxy 4-(1,4-benzodioxan-5-yl) pipéridine

A une solution préalablement refroidie à -60°C de 8 g de 5-bromo 1,4-benzodioxane dans 400 ml de tétrahydrofurane, on ajoute goutte à goutte 23,3 ml d'une solution de butyllithium 1,6 M dans l'hexane. L'addition términée, on agite à -60°C pendant 5 minutes, puis on ajoute goutte à goutte une solution de 8 g de 1-benzyl pipérid-4-one dans 100 ml de tétrahydrofurane. On agite le milieu 2 heures à -40°C puis 2 heures à température ambiante avant d'hydrolyser à l'aide de 80 ml d'eau.

La phase organique est décantée, la phase aqueuse est extraite par deux fois 100 ml de dichlorométhane. Les phases organiques réunies sont lavées une fois à l'aide de 20 ml d'eau, séchées sur sulfate de magnésium.

Les solvants sont évaporés sous vide et le produit obtenu au stade brut est chromatographié sur 520 g de silice (70 - 230 Mesh) en éluant à l'aide d'un mélange dichlorométhane - méthanol 98 : 2 (V/V).

– Rendement : 72 %

Spectre de résonance magnétique nucléaire du proton (solvant $CDCl_3$) : 2,05 ppm **m** 2H ; 2,1 ppm **m** 2H ; 2,55 ppm **m** 2H ; 2,75 ppm **m** 2H ; 3,6 ppm **s** 2H ; 4,25 ppm **m** 4H ; 6,8 ppm **m** 3H ; 7,1-7,4 ppm **m** 5H

**Stade B**

Une solution de 8 g du produit obtenu au stade précédent dans 100 ml d'acide acétique glacial et 8 ml d'acide chlorhydrique concentré est portée au reflux pendant 14 heures.

Après retour à la température ambiante, le milieu est concentré et le résidu repris à l'aide de 250 ml de dichlorométhane.

La phase organique décantée est lavée une fois à l'aide de 80 ml d'hydroxyde de sodium 1N puis une fois à l'aide de 80 ml d'eau.

Après séchage sur sulfate de magnésium et concentration sous vide, on obtient une huile.

– Rendement : 74 %

**EXEMPLE 23**

**Chlorhydrate de la 4-[5-(1,4-benzodioxan-5-yl)] 1-[2-(4-fluorobenzamido) éth-1-yl] 1,2,3,6-tétrahydropyridine**

**Stade A**

[4-(1,4-benzodioxan-5-yl) 1,2,3,6-tétrahydropyrid-1-yl] carbamate d'éthyle

A une solution contenant 5,5 g du produit de l'exemple 22 dans 50 ml de toluène, sont ajoutés sous agitation à température ambiante, 8 g de chloroformiate d'éthyle. L'addition terminée, on porte au reflux pendant 2 heures.

La solution refroidie est filtrée, concentrée sous vide et le résidu est repris dans 250 ml d'un mélange d'éther éthylique et d'éther diisopropylique 50 : 50 (V/V). Le précipité est filtré et le filtrat concentré pour obtenir une huile.

– Rendement : 63 %

Spectre de résonance magnétique nucléaire du proton (solvant $CDCl_3$) : 1,3 ppm **t** 3H ; 2,5 ppm **m** 2H ; 3,65 ppm **m** 2H ; 4-4,30 ppm **m** 2H ; 4,10 ppm **s** 4H ; 4,35 ppm **q** 2H ; 5,8 ppm **m** 1H ; 6,6-6,8 ppm **m** 3H

**Stade B**

Chlorhydrate de la 4-[(1,4-benzodioxan-5-yl)] 1,2,3,6-tétrahydropyridine

A une solution de 3 g du carbamate obtenu au stade précédent dans 4,2 ml de chloroforme, on ajoute goutte à goutte 1,7 ml d'iodure de triméthylsilane. L'addition terminée le milieu est agité 1 heure au reflux puis 2 heures à température ambiante.

Le milieu est rapidement filtré, le filtat dilué à l'aide de 20 ml de chloroforme, 10 ml de méthanol chlorhydrique sont alors additionnés goutte à goutte, et le milieu est dilué à l'aide de 30 ml d'éther éthylique. Le précipité formé est le produit attendu.

– Rendement : 77 %

– Point de fusion : 184°C (décomposition)

**Stade C**

[4-(1,4-benzodioxan-5-yl) 1,2,3,6-tétrahydropyrid-1-yl] acétonitrile

A une solution de 1,9 g de chlorhydrate de l'amine obtenue au stade B dans 40 ml d'acétone, on ajoute

2,5 g de carbonate de potassium et goutte à goutte une solution de 1,08 g de bromoacétonitrile dans 10 ml d'acétone.

Le milieu est agité 2 heures à température ambiante, filtré et concentré sous vide. Le résidu repris dans 20 ml de dichlorométhane est lavé à l'aide de 10 ml d'eau, puis la phase organique est séchée sur sulfate de magnésium.

Après évaporation du solvant, on obtient une huile.

– Rendement : 99 %

Spectre de résonance magnétique nucléaire du proton (solvant $CDCl_3$) : 2,6 ppm **m** 2H ; 2,8 ppm **t** 2H ; 3,3 ppm **m** 2H ; 3,65 ppm **s** 2H ; 4,2 ppm **s** 4H 5,8 ppm **m** 1H ; 6,75 ppm **m** 3H

**Stade D**

1-(2-aminoéthyl) 4-(1,4-benzodioxan-5-yl) 1,2,3,6-tétrahydropyridine

Une solution contenant 1, 9 g du composé obtenu au stade C, dans 20 ml de tétrahydrofurane anhydre, est ajoutée goutte à goutte à une suspension de 0,56 g d'hydrure de lithium et d'aluminium dans 30 ml de tétrahydrofurane anhydre.

Après 4 heures de contact à température ambiante, le milieu est hydrolysé à l'aide de 0,6 ml d'une solution saturée de chlorure d'ammonium.

Le milieu est filtré et le filtrat séché sur sulfate de magnésium.

Après concentration du solvant on obtient une huile qui est utilisée telle quelle dans l'étape suivante.

**Stade E**

La totalité du composé obtenu au stade D est mise en solution dans 20 ml de chloroforme. On y ajoute une solution de 0,64 g de triéthylamine dans 5 ml de chloroforme puis goutte à goutte, une solution de 0,94 g de chlorure de l'acide para-fluorobenzoïque dans 30 ml de chloroforme.

Après trois heures de contact, le milieu est lavé une fois à l'aide de 10 ml d'eau, la phase organique est séchée sur sulfate de magnésium, puis concentrée sous vide.

l'huile brute est solubilisée dans 50 ml d'éther et le produit est précipité par ajout d'une solution d'éther chlorhydrique.

Le précipité filtré est repris dans 20 ml d'éther. On otient ainsi un solide qui est le sel attendu.

– Rendement : 68 %

– Point de fusion : 214°C

**EXEMPLE 24**

**[4-(3,4-dihydro-7-méthoxy napht-1-yl) pipérid-1-yl] acétonitrile**

**Stade A**

[4-(3,4-dihydro-7-méthoxy napht-1-yl) pipérid-1-yl] carbamate d'éthyle

Le produit attendu est obtenu en utilisant le composé de l'exemple 15 et en le traitant selon le procédé décrit au stade A de l'exemple 3.

– Rendement : 93 %

**Stade B**

4-(3,4-dihydro-7-méthoxy napht-1-yl) pipéridine

Le produit attendu est obtenu en utilisant le composé décrit au stade A et en le traitant selon le procédé décrit au stade B de l'exemple 17.

– Rendement : 77 %

**Stade C**

[4-(3,4-dihydro-7-méthoxy napht-1-yl) pipérid-1-yl] acétonitrile

Le produit attendu est obtenu en utilisant le composé décrit au stade B et en le traitant selon le procédé décrit dans l'exemple 4.

– Rendement : 92 %
– Microanalyse élémentaire :

| | C % | H % | N % |
|---|---|---|---|
| calculé | 76,56 | 7,85 | 7,79 |
| trouvé | 75,50 | 9,92 | 9,57 |

**EXEMPLE 25**

**1-(2-aminoéth-1-yl)-4-(3,4-dihydro-7-méthoxy napht-1-yl) pipéridine**

Le produit attendu est obtenu en utilisant le composé de l'exemple 24 et en le traitant selon le procédé décrit au stade A de l'exemple 5.

– Rendement : 81 %

**EXEMPLE 26**

**1-(2-aminoéth-1-yl)-4-(1,2,3,4-tétrahydro-7-méthoxy napht-1-yl) pipéridine**

900 mg du composé décrit dans l'exemple 25 sont réduits par hydrogénation catalytique à température ambiante en présence de 0,1 g d'oxyde de platine dans 10 ml d'acide acétique. Le produit attendu est obtenu après filtration du catalyseur, neutralisation par de la soude et extraction au dichlorométhane.

– Rendement : ≃ 100 %
– Microanalyse élémentaire :

| | C % | H % | N % |
|---|---|---|---|
| calculé | 74,96 | 9,78 | 9,71 |
| trouvé | 74,99 | 9,83 | 9,27 |

**EXEMPLE 27**

**1-[2-(4-fluorobenzamido)éth-1-yl]-4-(3,4-dihydro-7-méthoxy napht-1-yl) pipéridine, chlorhydrate**

Le produit attendu est obtenu sous forme de base en utilisant le composé de l'exemple 25 et en le traitant selon le procédé décrit au stade B de l'exemple 5. La base est transformée en chlorhydrate correspondant par traitement par de l'alcool chlorhydrique.

– Rendement : 65 %
– Point de fusion : 250°C
– Microanalyse élémentaire :

| | C % | H % | N % | Cl % |
|---|---|---|---|---|
| calculé | 67,48 | 6,80 | 6,30 | 7,97 |
| trouvé | 67,36 | 6,78 | 6,23 | 7,95 |

**EXEMPLE 28**

**1-[2-(4-fluorobenzamido)éth-1-yl]-4-(1,2,3,4-tétrahydro-7-méthoxy napht-1-yl) pipéridine, chlorhydrate**

Le produit attendu est obtenu sous forme de base en utilisant le composé de l'exemple 26 et en le traitant selon le procédé décrit au stade B de l'exemple 5. La base est transformée en chlorhydrate correspondant par traitement par de l'alcool chlorhydrique.

– Rendement : 42 %
– Point de fusion : 204°C
– Microanalyse élémentaire

| | C % | H % | N % | Cl % |
|---|---|---|---|---|
| calculé | 67,18 | 7,22 | 6,27 | 7,93 |
| trouvé | 67,36 | 7,24 | 6,42 | 7,87 |

**EXEMPLE 29**

**1-benzyl-3-[(7-méthoxy napht-1-yl)méthyl] pyrrolidine**

64 mmoles du composé obtenu au stade B de l'exemple 13 sont portées à reflux dans 220 ml d'éthanol anhydre en présence de 19 g d'o-chloranyl et de 8,5 ml d'une solution éthanolique d'acide chlorhydrique 7,6 N.

Après évaporation, le résidu est repris par de l'eau, l'ensemble est amené à pH 10, filtré sur célite et extrait au dichlorométhane.

Le produit attendu est obtenu après séchage et évaporation des solvants et purifié par chromatographie sur silice (solvant d'élution : dichlorométhane/méthanol/ammoniaque : 99/1/0,1).

– Rendement : 70 %

**EXEMPLE 30**

**3-[(7-méthoxy napht-1-yl)méthyl] pyrrolidine**

Le produit attendu est obtenu par hydrogénation catalytique à 60°C du composé de l'exemple 29 dans de l'éthanol en présence de 1,5 g de palladium sur charbon.

– Rendement : 85 %

**EXEMPLE 31**

**1-cyanométhyl-3-[(7-méthoxy napht-1-yl)méthyl] pyrrolidine**

Le produit attendu est obtenu en utilisant le composé de l'exemple 30 et en le traitant selon le procédé décrit dans l'exemple 4 sans transformation en chlorhydrate.

– Rendement : 95 %

**EXEMPLE 32**

**1-(2-aminoéth-1-yl)-3-[(7-méthoxy napht-1-yl)méthyl] pyrrolidine**

Le produit attendu est obtenu en utilisant le composé de l'exemple 31 et en le traitant selon le procédé décrit au stade A de l'exemple 5.

– Rendement : 65 %

**EXEMPLE 33**

**1-[2-(4-fluorobenzamido)éth-1-yl]-3-[(7-méthoxy napht-1-yl)méthyl] pyrrolidine**

Le produit attendu est obtenu en utilisant le composé de l'exemple 32 et en le traitant selon le procédé décrit au stade B de l'exemple 5.

- Rendement : 80 %
- Point de fusion : > 260°C
- Microanalyse élémentaire

| | C % | H % | N % |
|---|---|---|---|
| calculé | 65,31 | 5,89 | 5,64 |
| trouvé | 64,99 | 5,72 | 5,99 |

TABLEAU I

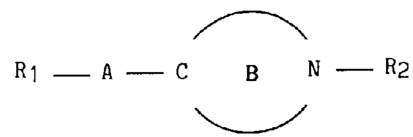

| EXEMPLE N° | $R_1$ | A | B | $R_2$ | SPECTRE RMN (Solvant) s = sel b = base |
|---|---|---|---|---|---|
| 1 | 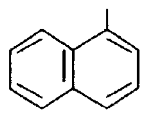 | — | 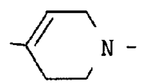 | $-CH_3$ | RMN-$^1$H (DMSO-$d_6$) **s** 2,75 ppm **m** 2H ; 2,95 ppm **s** 3H ; 3,5 ppm **m** 2H ; 3,95 ppm **m** 2H ; 5,75 ppm **m** 1H ; 7,35 ppm **dd** 1H ; 7,55 ppm **m** 3H ; 7,9 ppm **d** 1H ; 8 ppm **m** 1H ; 8,05 ppm **m** 1H ; 9,5-10,5 ppm **1H échangeable** |
| 2 | 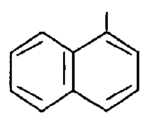 | — | 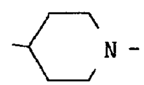 | $-CH_3$ | RMN-$^1$H (DMSO-$d_6$) **s** 2-2,2 ppm **m** 4H ; 2,8 ppm **s** 3H ; 3,3 ppm **m** 2H ; 3,6 ppm **m** 2H ; 3,7 ppm **m** 1H ; 7,3-7,7 ppm **m+d** 3H+1H ; 7,85 ppm **d** 1H ; 7,95 ppm **dd** 1H ; 8,2 ppm **dd** 1H ; 9,2-10,2 ppm **1H échangeable** |

TABLEAU I

suite 1

| EXEMPLE N° | R1 | A | B | R2 | SPECTRE RMN (Solvant) s = sel b = base |
|---|---|---|---|---|---|
| 3 | 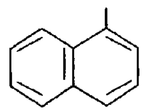 | — | 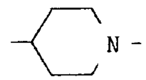 | H | RMN-$^1$H (DMSO-d6) **s** 1,9-2,15 ppm **m** 4H ; 3,1-3,6 ppm **m** 4H ; 3,8 ppm **m** 1H ; 7,3-7,65 ppm **m+t+d** 2H+1H+1H ; 7,8 ppm **d** 1H ; 7,95 ppm **m** 1H 8,3 ppm **m** 1H ; 8,4-9 ppm **1H échangeable** |
| 4 | 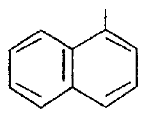 | — | 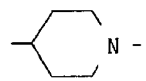 | $-CH_2CN$ | RMN-$^1$H (DMSO-d6) **s** 2-2,4 ppm **m** 4H ; 3,2-3,9 ppm **m+m+m** 1H+2H+2H ; 4,6 ppm **s** 2H 7,25-7,65 ppm **m+d** 3H+1H ; 7,8 ppm **d** 1H ; 7,9 ppm **dd** 1H ; 8,25 ppm **dd** 1H |
| 5 | 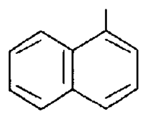 | — | 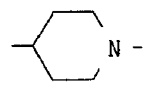 | 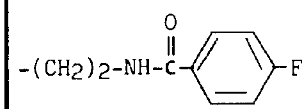 | RMN-$^1$H (DMSO-d6) **s** 1,8-2,4 ppm **m** 4H ; 3-3,5 ppm **m** 4H ; 3,5-4 ppm **m** 5H ; 7,2-7,7 ppm **m+m+t** 3H+1H+2H ; 7,8 ppm **d** 1H ; 7,85-8,15 ppm **dd+m** 2H+1H ; 8,2 ppm **d** 1H ; 8,8-9,3 ppm **1H échangeable** ; 10,4-10,7 ppm 1H **échangeable** |

**TABLEAU I**

suite 2

| EXEMPLE N° | $R_1$ | A | B | $R_2$ | SPECTRE RMN (Solvant) s = sel b = base |
|---|---|---|---|---|---|
| 6 | 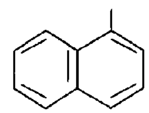 | — | 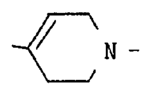 | $-(CH_2)_2-NH-$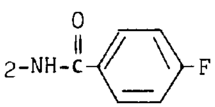 | RMN-$^1H$ (DMSO-$d_6$) **s** 2,5-2,7 ppm **m** 1H ; 2,9-3,2 ppm **m** 1H ; 3,3-3,6 ppm **m+m** 2H 3,7-4,05 ppm **m+t** 2H+2H ; 4,2 ppm **m** 1H ; 5,75 ppm **m** 1H 7,25-7,40 ppm **m** 3H ; 7,45-7,60 ppm **m** 3H ; 7,8-8,0 ppm **m+m** 1H+1H ; 8,05-8,3 ppm **m+dd** 1H+2H ; 9,15 ppm **1H échangeable** ; 11-11,4 ppm **1H échangeable** |
| 7 | 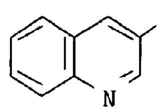 | — | 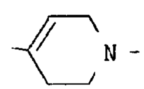 | $-CH_3$ | RMN-$^1H$ (DMSO-$d_6$) **s** 3,0 ppm **m+s** 5H ; 3,4-3,75 ppm **m** 2H ; 4,0-4,2 ppm **m** 2H ; 6,8 ppm **m** 1H ; 7,9 ppm **m** 1H ; 8,05 ppm **m** 1H ; 8,25 ppm **d** 1H 8,3 ppm **m** 1H ; 9,1 ppm **d** 1H ; 9,5 ppm **d** 1H ; 5,5-7 **1H échangeable** ; 9,5-10,5 ppm **1H échangeable** |

TABLEAU I

suite 3

| EXEMPLE N° | $R_1$ | A | B | $R_2$ | SPECTRE RMN (Solvant) s = sel b = base |
|---|---|---|---|---|---|
| 8 | 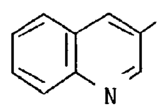 | — | 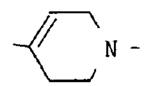 | -H | RMN-$^1H$ (DMSO-$d_6$) s 2,9 ppm **m** 2H ; 3,4-3,75 ppm **m** 2H ; 4,0-4,2 ppm **m** 2H ; 6,8 ppm **m** 1H ; 7,9 ppm **m** 1H ; 8,05 ppm **m** 1H ; 8,25 ppm **d** 1H 8,3 ppm **m** 1H ; 9,1 ppm **d** 1H ; 9,5 ppm **d** 1H ; 5,5-7 **1H échangeable** ; 9,5-10,5 ppm **1H échangeable** |
| 9 | 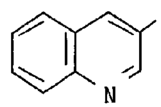 | — | 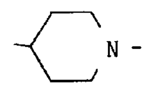 | $-CH_3$ | RMN-$^1H$ (DMSO-$d_6$) s 2,10 ppm **m** 2H ; 2,25 ppm **m** 2H 2,85 ppm **s** 3H ; 3,20 ppm **m** 2H 3,30 ppm **m** 1H ; 3,60 ppm **m** 2H 7,95 ppm **m** 1H ; 8,10 ppm **m** 1H 8,25 ppm **d** 1H ; 8,35 ppm **d** 1H 9,00 ppm **d** 1H ; 9,30 ppm **d** 1H 1,7-4 ppm **1H échangeable** 9-10,2 ppm **1H échangeable** |
| 10 | 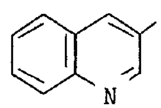 | — | 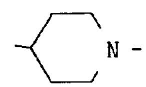 | -H | RMN-$^1H$ (DMSO-$d_6$) s 2,05 ppm **m** 2H ; 2,25 ppm **m** 2H 3,15 ppm **m** 2H ; 3,35 ppm **m** 1H 3,50 ppm **m** 2H ; 7,95 ppm **m** 1H 8,10 ppm **m** 1H ; 8,25 ppm **d** 1H 8,35 ppm **d** 1H ; 9,15 ppm **d** 1H 9,30 ppm **d** 1H ; 5,5-9 ppm **2H échangeables** |

**TABLEAU I**
**suite 4**

| EXEMPLE N° | $R_1$ | A | B | $R_2$ | SPECTRE RMN (Solvant) s = sel b = base |
|---|---|---|---|---|---|
| 11 | 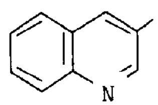 | — | 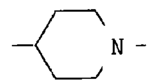 | $-CH_2CN$ | RMN-$^1$H ($D_2O$) s 2,1-2,4 ppm **m** 2H ; 2,45 ppm **m** 2H ; 3,35-3,6 ppm **m** 1H ; 3,45 ppm **m** 2H ; 3,85 ppm **m** 2H ; 4,55 ppm **s** 2H ; 7,85-8,3 ppm **d+d+t+t** 1H+1H+1H+1H ; 9,10 ppm **d** 1H ; 9,15 ppm **d** 1H |
| 12 | 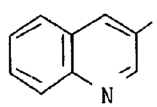 | — | 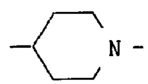 | 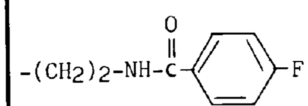 | RMN-$^1$H (DMSO-$d_6$) s 2,1-2,4 ppm **m** 4H ; 3,05-3,50 ppm **m** 5H ; 3,6-3,9 ppm **m** 4H ; 7,3 ppm **t** 2H ; 7,90 ppm **td** 1H 7,95-8,15 ppm **m** 3H ; 8,3 ppm **m** 2H ; 8,85 ppm **d** 1H ; 9,1 ppm **1H échangeable** ; 9,2 ppm **d** 1H ; 10,7-11,0 ppm **1H échangeable** |
| 13 | 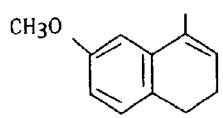 | $-CH_2-$ | 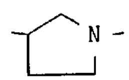 | 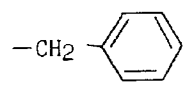 | RMN-$^1$H ($CDCl_3$) b 1,55 ppm **m** 1H ; 2,0 ppm **m** 2H 2,25 ppm **m** 2H ; 2,6-2,3 ppm **m** 5H ; 2,7 ppm **t** 2H ; 2,8 ppm **m** 1H ; 3,7-3,5 ppm **2d** 2H ; 3,8 ppm **s** 3H ; 5,85 ppm **t** 1H ; 6,6 ppm **dd** 1H ; 6,8 ppm **d** 1H 7,05 ppm **d** 1H ; 7,4-7,2 **m** 5H |

TABLEAU I

suite 5

| EXEMPLE N° | $R_1$ | A | B | $R_2$ | SPECTRE RMN (Solvant) s = sel b = base |
|---|---|---|---|---|---|
| 14 | 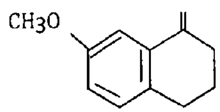 | = CH- | 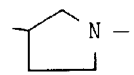 | 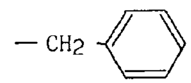 | RMN-$^1$H ($CDCl_3$) **b** 1,55 ppm **m** 1H ; 1,8 ppm **m** 1H 2 ppm **m** 1H ; 2,6-2,3 ppm **m+t+m** 2H+2H+2H ; 2,9 ppm **t** 2H 3,2 ppm **m** 1H ; 3,7-3,5 ppm **2d** 2H ; 3,8 ppm **s** 3H ; 5,95 ppm **dd** 1H ; 6,75 ppm **dd** 1H ; 6,95 ppm **d** 1H ; 7,15 ppm **d** 1H ; 7,4-7,2 ppm **m** 5H |
| 15 | 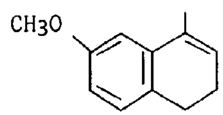 | — | 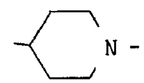 | $-CH_3$ | RMN-$^1$H ($CDCl_3$) **b** 1,6 ppm **m** 2H ; 1,85 ppm **m** 2H 2,1-2,2 ppm **m** 4H ; 2,3 ppm **s** 3H ; 2,45 ppm **m** 1H ; 2,65 ppm **m** 2H ; 3,00 ppm **m** 2H ; 3,8 ppm **s** 3H ; 5,9 ppm **m** 1H ; 6,7 ppm **dd** 1H ; 6,8 ppm **d** 1H ; 7,1 ppm **d** 1H |
| 16 | 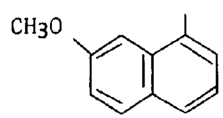 | — | 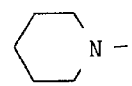 | $-CH_3$ | RMN-$^1$H ($CDCl_3$) **b** 1,8-2,1 ppm **m** 4H ; 2,2 ppm **m** 2H ; 2,4 ppm **s** 3H ; 3-3,3 ppm **m** 3H ; 3,95 ppm **s** 3H ; 7,15 ppm **dd** 1H ; 7,2-7,45 ppm **m** 3H 7,9 ppm **d** 1H ; 7,8 ppm **d** 1H |

TABLEAU I

suite 6

| EXEMPLE N° | $R_1$ | A | B | $R_2$ | SPECTRE RMN (Solvant) s = sel b = base |
|---|---|---|---|---|---|
| 17 | 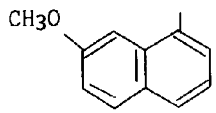 | — | 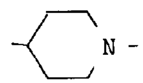 | -H | RMN-$^1$H (DMSO-$d_6$) s 1,8-2,1 ppm m 4H ; 3,1-3,5 ppm m 4H ; 3,5-3,8 ppm m 1H ; 3,9 ppm s 3H ; 7,2 ppm dd 1H 7,3 ppm m 2H ; 7,45 ppm d 1H 7,75 ppm m 1H ; 7,85 ppm d 1H 9-9,5 ppm 2H échangeables |
| 18 | 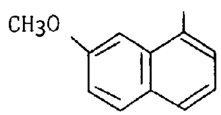 | — | 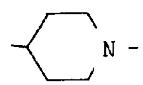 | 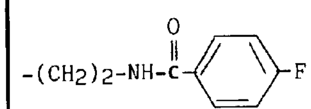 | RMN-$^1$H (DMSO-$d_6$) s 1,9-2,4 ppm m 4H ; 3,1-3,9 ppm m 9H ; 3,9 ppm s 3H ; 7,1-7,4 ppm m+t+d+dd 2H+1H +1H+1H ; 7,45 ppm d 1H ; 7,7 ppm m 1H ; 7,85 ppm d 1H ; 8,05 ppm dd 2H ; 9-10,7 ppm 2H échangeables |
| 19 | 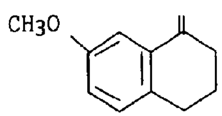 | = | 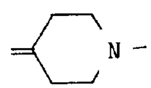 | -H | RMN-$^1$H (DMSO-$d_6$) s 1,7 ppm m 2H ; 2,3-2,6 ppm t+t 2H+2H ; 2,6 ppm t 2H ; 2,75 ppm t 2H ; 3,1 ppm m 2H 3,4 ppm m 2H ; 3,75 ppm s 3H 6,65 ppm d 1H ; 6,7 ppm dd 1H 7,05 ppm d 1H ; 9,3 ppm 1H échangeable |

TABLEAU I

suite 7

| EXEMPLE N° | $R_1$ | A | B | $R_2$ | SPECTRE RMN (Solvant) s = sel b = base |
|---|---|---|---|---|---|
| 20 | 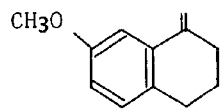 | = | 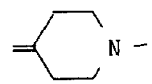 | $-CH_2CN$ | RMN-$^1H$ ($CDCl_3$) **b** 1,85 ppm <u>q</u> 2H ; 2,5 ppm <u>t</u> 2H 2,5-2,7 ppm <u>m</u> 8H ; 2,75 ppm <u>t</u> 2H ; 3,55 ppm <u>d</u> 2H ; 3,8 ppm <u>s</u> 3H ; 6,7 ppm <u>m</u> 2H ; 7,05 ppm <u>d</u> 1H |
| 21 | 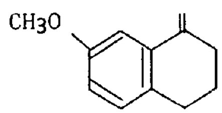 | = | 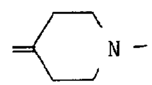 | 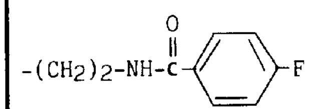 | RMN-$^1H$ ($CDCl_3$) **b** 1,2 ppm <u>q</u> 2H ; 2,3-2,8 ppm <u>m</u> 14H ; 3,55 ppm <u>q</u> 2H ; 3,8 ppm <u>s</u> 3H ; 6,75 ppm <u>m</u> 2H ; 7,0-7,3 <u>m</u> 3H ; 7,85 ppm <u>dd</u> 2H |
| 22 | 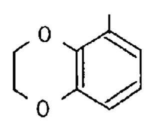 | — | 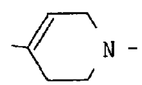 | 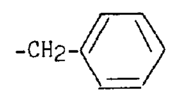 | RMN-$^1H$ ($CDCl_3$) **b** 2,8-2,4 ppm <u>m</u> 4H ; 3,1 ppm <u>m</u> 2H ; 3,6 ppm <u>s</u> 2H ; 4,2 ppm <u>s</u> 4H ; 5,8 ppm <u>m</u> 1H ; 6,75 ppm <u>m</u> 3H ; 7,3 ppm <u>m</u> 5H |
| 23 | 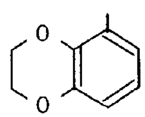 | — | 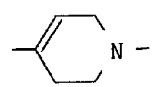 | 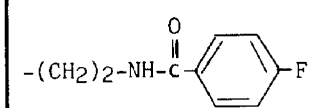 | RMN-$^1H$ ($CDCl_3$) **b** 2,7-3,8 ppm <u>m</u> 6H ; 3,8-4,25 ppm <u>m</u> 4H ; 4,25 ppm <u>m</u> 4H ; 5,8 ppm <u>m</u> 1H ; 6,65-6,9 ppm <u>m</u> 3H ; 7,15 ppm <u>t</u> 2H ; 8,2 ppm <u>dd</u> 2H ; 9 et 12,2 ppm 2H **échangeables** |

## ETUDE PHARMACOLOGIQUE

### EXEMPLE 34

### Evaluation de l'activité antihypertensive des composés de l'invention

Des chiens batards (mâles et femelles) ont été anesthésiés au phénobarbital (30 mg/kg i.v.) puis placés en respiration artificielle (respirateur Bird Mark VII). La pression artérielle à été mesurée au moyen d'un cathéther placé dans l'aorte abdominale via l'artère fémorale. Ce cathéter est connecté à une cellule de pression (Statham ® $R_{23}D_6$) reliée à un enregistreur.

La fréquence cardiaque a été mesurée au moyen d'un Gould Biotach ®.

L'activité nerveuse sympathique est enregistrée au niveau du nerf rénal au moyen d'une électrode d'argent. Le signal amplifié a été visualisé sur un oscilloscope (Tektronix 5115 ®) puis mesuré en µV au moyen d'un intégrateur Gould. Les composés à examiner ont été administrés par voie i.v.

Les résultats de cette étude indiqués dans le Tableau II ont démontré que les composés de l'invention sont plus actifs ou au moins comparables au produit de référence flesinoxan, sous forme racémique ou sous forme d'isomère (+). Cet isomère est l'isomère le plus actif du flesinoxan.

TABLEAU II

| COMPOSES | EFFET SUR LA PRESSION ARTERIELLE (mmHg) | EFFET SUR LE RYTHME CARDIAQUE (Batt/min.) | DOSES µg/kg |
|---|---|---|---|
| Flesinoxan Isomère (+) | ↘ # 10<br>↘ 10-30<br>↘ > 30 | ↘ # 15<br>↘ 15-30<br>↘ > 40 | 10<br>30<br>100 |
| Flesinoxan racémique | 0<br>↘ # 10<br>↘ 10-30<br>↘ > 30 | 0<br>0<br>↘ # 15<br>↘ 15-35 | 10<br>30<br>100<br>300 |
| Composés de l'invention | ↘ # 10<br>↘ 10-30<br>↘ > 30 | ↘ # 15<br>↘ 15-30<br>↘ 30-40 | 3-10<br>10-30<br>30-100 |

Concernant l'activité nerveuse sympathique, les résultats obtenus avec le composé de l'exemple 18 après administration d'une dose de 30 µg/kg par voie i.v., sont donnés dans la Figure 1.

### EXEMPLE 35

### Evaluation de l'affinité pour les récepteurs 5-$HT_{1A}$

Pour les essais, on a utilisé du tissu de l'hippocampe obtenu à partir de rats-Wistar décapités. Les animaux ont été sacrifiés 48 heures avant l'expérience et les tissus isolés ont été conservés à -86°C. Pour la préparation des membranes, les tissus ont été ensuite homogénisés en utilisant 20 volumes d'une solution tampon 50 mM

Tris HCl (pH = 7,7 ajusté à l'aide de $NH_4Cl$ à 25°C) pour un volume de tissus, à une température voisine de 0°C, avec un homogénisateur Polytron ®, puis, le tout a été centrifugé. (35 000 g x 20 min à 4°C). Le culot ainsi obtenu a été suspendu dans 100 volumes d'une solution tampon d'incubation. (Tris 60 mM, pargyline 10 μM, $CaCl_2$ 4 mM et acide ascorbique 0,1 % (p/v); pH ajusté à 7,7 avec HCl 5N). Les composés à examiner ont été aussi dilués dans le tampon d'incubation puis les solutions essais ont été préparées en ajoutant dans des tubes de verre de 12 x 75 mm, 100 μl d'une solution du composé à examiner, 100 μl d'une solution de [3H] 8-OH-DPAT C = 0,4 nm (radioactivité spécifique = 205 Ci/mmol). Le binding non spécifique a été déterminé à l'aide d'une solution de 5-hydroxytryptamine 10 μM et correspond à 5-10 % du binding total.

Les tubes ont été incubés pendant 30 min à 37°C, et les solutions ont été ensuite filtrées sur des filtres en fibres de verre GF/B traités avec 0,1 % de polyéthylèneimine (Whatman ®). Les filtres ont été rincés 2 fois avec 5 ml de la solution tampon d'incubation puis, ils ont été placés dans des ampoules dans lesquelles ont été ajoutés 4,5 ml de "Picofluor scintillation fluid" ® (Packard). La radioactivité à été détérminée en utlisant des standards externes.

Les pKi ont été évalués en utilisant l'équation de Cheung-Prusoff :

$$-\log (IC_{50} / [1 + [3H]\ 8\text{-OH - DPAT}] / Kd).$$

Les composés de l'invention ont une grande affinité pour les sites $5\text{-}HT_{1A}$. Les pKi des composés de l'invention sont de l'ordre de 9,02 moles/ litre.

**PREPARATION PHARMACEUTIQUE**

**EXEMPLE 36**

**Gélules dosées à 1 mg de chlorhydrate de la 1-[2-(4-fluorobenzamido) éth-1-yl] 4-(7-méthoxy napht-1-yl) pipéridine [F.A.E.M.N.P]**

| | |
|---|---|
| F.A.E.M.N.P. | 1 mg |
| Amidon de maïs | 15 mg |
| Lactose | 25 mg |
| Talc | 5 mg |

## Revendications

1. Composés de formule I :

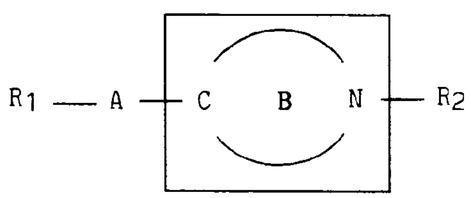

(I)

dans laquelle

- $R_1$ représente un radical naphtyle, de formule $y_1$ :

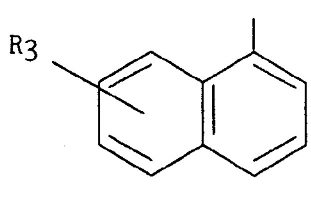

($y_1$)

un radical dihydronaphtyle, de formule $y_2$ :

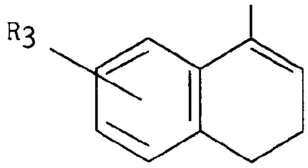

(y2)

un radical tétrahydronaphtyle de formule $y_3$ :

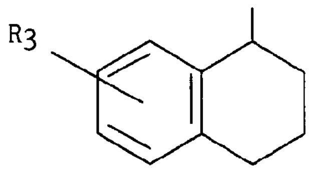

(y3)

(dans lesquelles $R_3$ représente un atome d'hydrogène, un atome d'halogène, un radical alkyle de 1 à 6 atomes de carbone, un radical hydroxy ou un radical alcoxy de 1 à 6 atomes de carbone), un radical quinol-3-yle (éventuellement substitué sur le cycle benzénique par un ou plusieurs atomes d'halogène, radicaux alkyle de 1 à 6 atomes de carbone, radicaux hydroxy, ou radicaux alcoxy de 1 à 6 atomes de carbone), ou un radical 1,4-benzodioxan-5-yl,

- A représente une simple liaison, une double liaison, (à condition toutefois que $R_1$ représente un radical tétrahydronaphtyle), un radical méthylène, un radical de formule $z_1$ :

$$—CH=\qquad (z_1)$$

ou un radical de formule $z_2$ :

$$=CH—\qquad (z_2)$$

(à condition toutefois que dans ce cas, $R_1$ représente un radical tétrahydronaphtyle),

- le cycle B représente un radical pipéridyle (à condition toutefois que dans ce cas A représente une simple ou une double liaison), un radical pyrrolidinyle (à condition toutefois que dans ce cas A représente un radical méthylène, un radical $z_1$ ou un radical $z_2$), ou un radical 1,2,3,6-tétrahydropyridyle (à condition toutefois que dans ce cas A représente une simple liaison),

$R_2$ représente :

– un atome d'hydrogène, un radical benzyle, un radical alkyle de 1 à 6 atomes de carbone, à condition que dans l'un de ces cas $R_1$ soit différent d'un radical naphtyle ($y_1$), dihydronaphtyle ($y_2$) ou tétrahydronaphtyle ($y_3$) et B soit différent d'un radical pipéridinyl,

– un radical aminoalkyle de 1 à 6 atomes de carbone, un radical cyanoalkyle de 1 à 6 atomes de carbone ou un radical de formule $w_1$ :

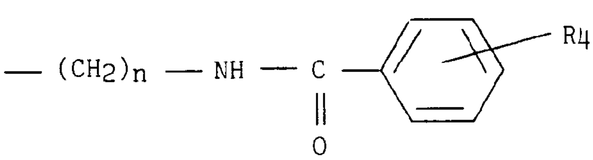

($w_1$)

(dans laquelle :

n est égal à 1-6

et

$R_4$ représente un atome d'hydrogène, un atome d'halogène, un radical alkyle de 1 à 6 atomes de carbone ou un radical alcoxy de 1 à 6 atomes de carbone),

à condition toutefois que

– quand $R_1$ est un radical $y_2$, $R_3$ représente un atome d'hydrogène, A une simple liaison et B un radical

pipéridyle, $R_2$ ne représente pas simultanément un radical méthyle,
et
– quand $R_2$ est un radical $y_1$, $R_3$ représente un atome d'hydrogène, A une simple liaison et B un radical 1,2,3,6-tétrahydropyridyle, $R_2$ ne représente pas simultanément un atome d'hydrogène,
et
– quand $R_1$ est un radical $y_3$, $R_3$ représente un atome d'hydrogène, A une double liaison et B un radical pipéridyle, $R_2$ ne représente pas simultanément un radical méthyle,
**leurs stéréoisomères possibles,**
**et leurs sels d'addition** à un acide minéral ou organique pharmaceutiquement acceptable.

**2.** La 1-[2-(4-fluorobenzamido) éth-1-yl] 4-(7-méthoxynapht-1-yl) pipéridine, composé répondant à la formule I selon la revendication 1 et ses sels d'addition à un acide minéral ou organique, pharmaceutiquement acceptable.

**3.** Procédé de préparation des composés de formule générale I, selon la revendication 1, caractérisé en ce que
l'on fait réagir un composé de formule II :

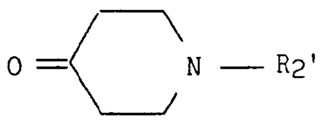

(II)

dans laquelle $R_{2'}$ représente un radical alkyle de 1 à 6 atomes de carbone, ou un radical benzyle, avec un composé de formule III :

$$R_1 — X \quad (III)$$

dans laquelle $R_1$ a la même signification que pour la formule I et X représente un atome de brome, de lithium ou un groupement MgBr, selon la revendication 1, pour former les composés de formule IV :

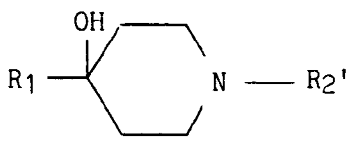

(IV)

dans laquelle $R_1$ a la signification indiquée pour la formule I, selon la revendication 1, et $R_{2'}$ représente un radical alkyle de 1 à 6 atomes de carbone ou un radical benzyle,
que l'on soumet à l'action de l'acide bromhydrique pour former les composés de formule $I_a$ :

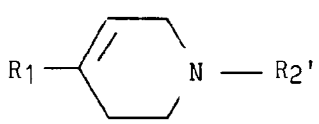

($I_a$)

dans laquelle $R_1$ a la même signification que pour la formule I, selon la revendication 1, et $R_{2'}$ représente un radical alkyle de 1 à 6 atomes de carbone ou un radical benzyle,
et ensuite
- soit
les composés de formule $I_a$ sont soumis à l'action du chloroformiate d'éthyle en présence d'un sel minéral alcalin pour former les composés de formule V :

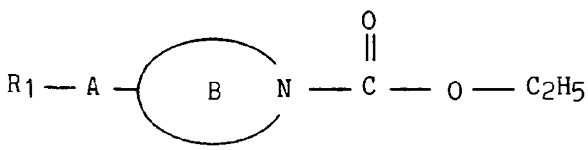

(V)

dans laquelle $R_1$ a la même signification que pour la formule I, selon la revendication 1, A est une simple liaison et B représente un radical 1,2,3,6-tétrahydropyridyle,

- soit

les composés de formule $I_a$ sont d'abord soumis à une hydrogénation catalytique pour former les composés de formule $I_b$ :

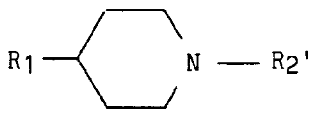

($I_b$)

dans laquelle $R_1$ a la même signification que pour la formule I, selon la revendication 1, et $R_{2'}$ représente un radical alkyle de 1 à 6 atomes de carbone ou un radical benzyle,

puis, à l'action du chloroformiate d'éthyle en présence d'un sel minéral alcalin pour former les composés de formule V,

dans laquelle $R_1$ a la même signification que pour la formule I, selon la revendication 1, et B représente un radical pipéridyle,

dérivés de formule (Ia) et (Ib) que l'on sépare en leurs stéréoisomères possibles, et/ou que l'on salifie avec un acide organique ou minéral, pharmaceutiquement acceptable, pour former les sels d'addition correspondants.

**4.** Procédé de préparation de composés de formule générale I, selon la revendication 1, caractérisé en ce que l'on fait réagir un composé de formule VI :

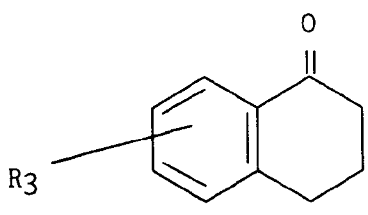

(VI)

dans laquelle $R_3$ a la même signification que pour la formule I, selon la revendication 1,

ou

avec un composé de formule VII,

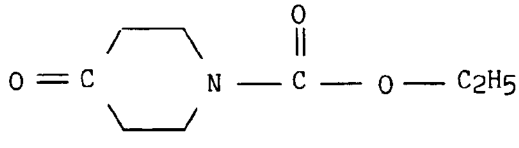

(VII)

pour former les composés de formule V:

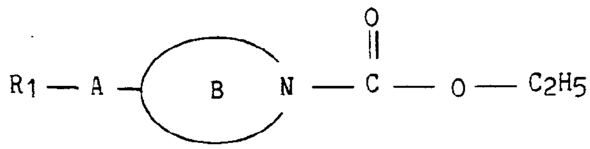

(V)

dans laquelle $R_1$ représente un radical tétrahydronaphtyle de formule $y_3$, A est une double liaison et B représente un radical pipéridyle,

<u>ou</u>

avec un composé de formule VIII :

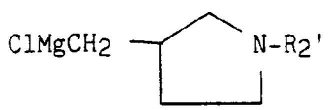

(VIII)

dans laquelle $R_{2'}$ a la même signification que pour la formule II, pour former les composés de formule IX :

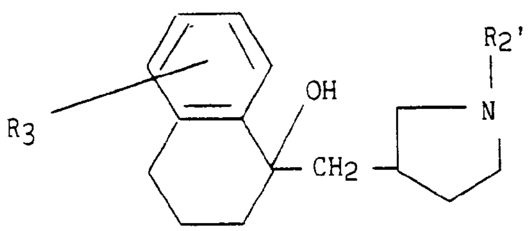

(IX)

dans laquelle $R_3$ à la même signification que pour la formule I, selon la revendication 1, et $R_{2'}$ représente un radical alkyle de 1 à 6 atomes de carbone ou un radical benzyle,

que l'on soumet à l'action de l'acide para-toluènesulfonique pour former les composés de formule $I_c$ et $I_d$ :

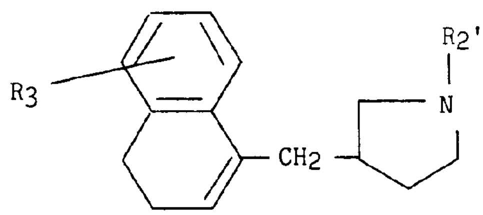

($I_c$)

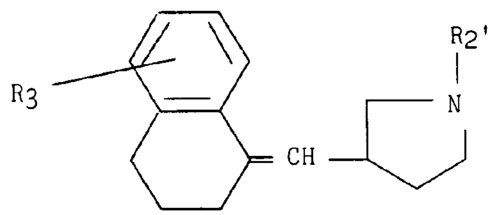

(Id)

dans lesquelles $R_3$ a la même signification que pour la formule I, selon la revendication 1, et la signi-

fication de $R_{2'}$ est identique à celle donnée pour la formule II, lesquels ensuite sont séparés

puis soumis à l'action du chloroformiate d'éthyle en présence d'un sel minéral alcalin pour former les composés de formule V, dans laquelle $R_1$ représente un radical dihydronaphtyle de formule $y_2$, selon la revendication 1, ou un radical tétrahydronaphtyle de formule $y_3$, selon la revendication 1, A est respectivement un radical méthylène ou un radical de formule $z_2$ et B représente un radical pyrrolidinyle,

<u>ou</u>

avec un composé de formule X :

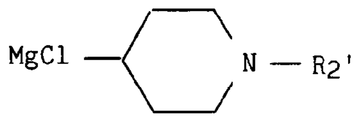

(X)

dans laquelle $R_{2'}$ a la même signification que pour la formule II, pour former les composés de formule XI :

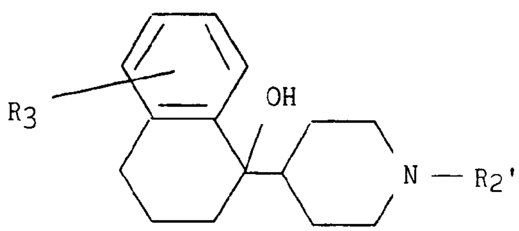

(XI)

dans laquelle la signification de $R_3$ reste identique à celle donnée pour la formule I, selon la revendication 1, et $R_{2'}$ représente un radical benzyle ou un radical alkyle de 1 à 6 atomes de carbone,

lesquels ensuite sont soumis à l'action de l'acide paratoluènesulfonique pour former les composés de formule $I_e$ :

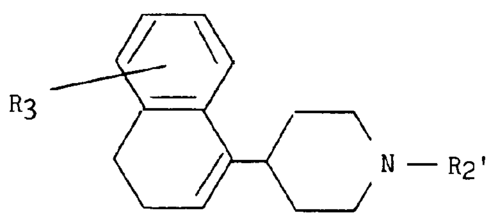

($I_e$)

dans laquelle $R_3$ a la même signification que pour la formule I, selon la revendication 1, et $R_{2'}$ représente un radical alkyle de 1 à 6 atomes de carbone ou un radical benzyle,

lesquels ensuite

- soit

l'on fait réagir avec le chloroformiate d'éthyle pour former les composés de formule V dans laquelle $R_1$ représente un radical dihydronaphtyle de formule $y_2$ selon la revendication 1, A est une simple liaison et B représente un radical pipéridyle,

- soit

l'on soumet à l'action de tétrachloro 1,2-benzoquinone, pour former les composés de formule $I_f$ :

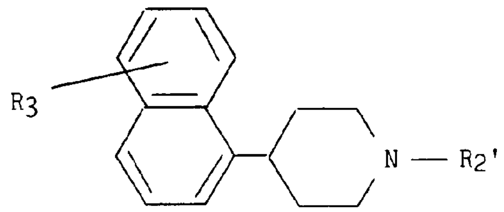

($I_f$)

dans laquelle $R_3$ a la même signification que pour la formule I, selon la revendication 1, et $R_{2'}$ représente un radical alkyle de 1 à 6 atomes de carbone ou un radical benzyle,

lesquels ensuite

l'on fait réagir avec le chloroformiate d'éthyle pour former les composés de formule V dans laquelle $R_1$ représente un radical naphtyle de formule $y_1$, A est une simple liaison et B représente un radical pipéridyle,

et ensuite

l'on soumet les composés de formule V, soit à l'action de l'acide bromhydrique, soit à l'action de l'hydroxyde de potassium, pour former les composés de formule $I_g$ :

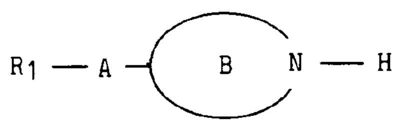

($I_g$)

dans laquelle la signification de $R_1$, A et B reste identique à celle donnée pour la formule I, selon la revendication 1,

lesquels ensuite l'on fait réagir avec un composé de formule XII :

$$Br-(CH_2)_{n-1}-CN \quad (XII)$$

dans laquelle n a la même signification que pour la formule I, selon la revendication 1, pour former les composés de formule $I_h$ :

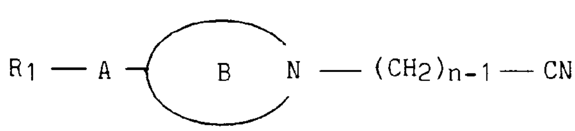

($I_h$)

dans laquelle $R_1$, A, B et n ont la même signification que pour la formule I, selon la revendication 1,

lesquels ensuite sont soumis à l'action d'hydrure de lithium et d'aluminium pour former les composés de formule $I_i$ :

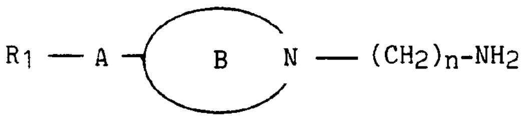

$(I_i)$

dans laquelle la signification de $R_1$, A, B et n reste identique à celle donnée pour la formule I, selon la revendication 1,
que l'on fait réagir avec un composé de formule XIII :

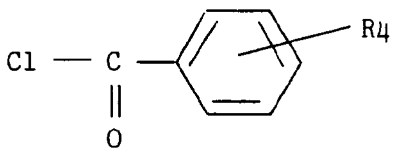

(XIII)

dans laquelle la signification de $R_4$ reste identique à celle donnée pour la formule I, selon la revendication 1,
pour former les composés de formule $I_j$ :

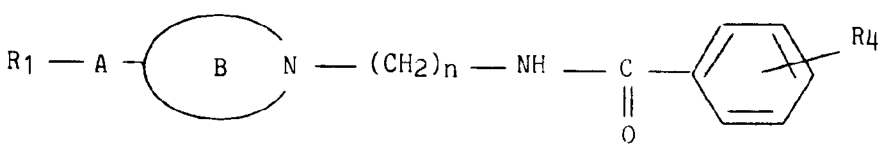

$(I_j)$

dans laquelle $R_1$, A, B, n et $R_4$ ont la même signification que pour la formule I, selon la revendication 1,

et ensuite

l'on sépare les composés de formule $I_c$-$I_j$ en leurs stéréoisomères possibles,
et/ou l'on salifie avec un acide organique ou minéral, pharmaceutiquement acceptable, pour former les sels d'addition correspondants.

5. Composition pharmaceutique contenant comme principe actif un composé selon l'une quelconque des revendications 1 à 2, en association ou en mélange avec un excipient ou un véhicule inerte non toxique, pharmaceutiquement acceptable.

6. Composition pharmaceutique selon la revendication 4 renfermant le principe actif à la dose de 0,1 à 100 mg.

7. Composition pharmaceutique selon les revendications 4 et 5 renfermant comme principe actif au moins un composé selon les revendications 1 à 2 utilisable dans le traitement des maladies nécessitant des ligands aux récepteurs 5-$HT_{1a}$.

8. Composition pharmaceutique selon les revendications 4 et 5 renfermant comme principe actif au moins

un composé selon les revendications 1 à 2 utilisable dans le traitement de l'hypertension.

**Revendications pour l'état contractant suivant: ES**

**1.** Procédé de préparation des composés de formule I :

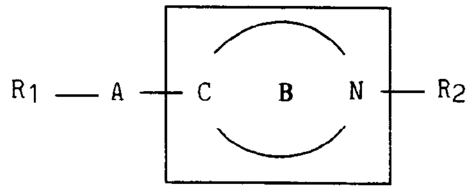

(I)

dans laquelle

- $R_1$ représente un radical naphtyle, de formule $y_1$ :

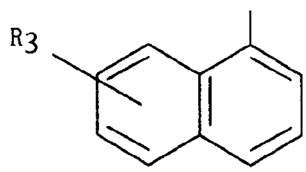

($y_1$)

un radical dihydronaphtyle, de formule $y_2$

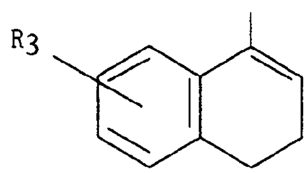

($y_2$)

un radical tétrahydronaphtyle de formule $y_3$ :

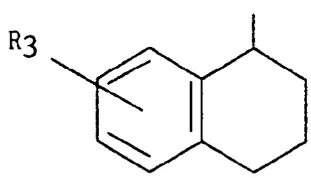

($y_3$)

(dans lesquelles $R_3$ représente un atome d'hydrogène, un atome d'halogène, un radical alkyle de 1 à 6 atomes de carbone, un radical hydroxy ou un radical alcoxy de 1 à 6 atomes de carbone), un radical quinol-3-yle (éventuellement substitué sur le cycle benzénique par un ou plusieurs atomes d'halogène, radicaux alkyle de 1 à 6 atomes de carbone, radicaux hydroxy, ou radicaux alcoxy de 1 à 6 atomes de carbone), ou un radical 1,4-benzodioxan-5-yl,

- A représente une simple liaison, une double liaison, (à condition toutefois que $R_1$ représente un radical tétrahydronaphtyle), un radical méthylène, un radical de formule $z_1$ :

$$-CH= \qquad (z_1)$$

ou un radical de formule $z_2$ :

$$=CH- \qquad (z_2)$$

(à condition toutefois que dans ce cas, $R_1$ représente un radical tétrahydronaphtyle),

- le cycle B représente un radical pipéridyle (à condition toutefois que dans ce cas A représente une simple ou une double liaison), un radical pyrrolidinyle (à condition toutefois que dans ce cas A représente un radical méthylène, un radical $z_1$ ou un radical $z_2$), ou un radical 1,2,3,6-tétrahydropyridyle (à condition toutefois que dans ce cas A représente une simple liaison),

$R_2$ représente :

– un atome d'hydrogène, un radical benzyle, un radical alkyle de 1 à 6 atomes de carbone, à condition que dans l'un de ces cas $R_1$ soit différent d'un radical naphtyle ($y_1$), dihydronaphtyle ($y_2$) ou tétrahydronaphtyle ($y_3$) et B soit différent d'un radical pipéridinyl,

– un radical aminoalkyle de 1 à 6 atomes de carbone, un radical cyanoalkyle de 1 à 6 atomes de carbone ou un radical de formule $w_1$ :

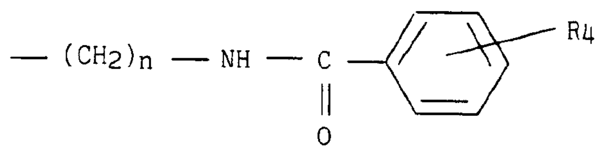

($w_1$)

(dans laquelle

n est égal à 1-6

et

$R_4$ représente un atome d'hydrogène, un atome d'halogène, un radical alkyle de 1 à 6 atomes de carbone ou un radical alcoxy de 1 à 6 atomes de carbone),

à condition toutefois que

– quand $R_1$ est un radical $y_2$, $R_3$ représente un atome d'hydrogène, A une simple liaison et B un radical pipéridyle, $R_2$ ne représente pas simultanément un radical méthyle,

et

– quand $R_2$ est un radical $y_1$, $R_3$ représente un atome d'hydrogène, A une simple liaison et B un radical 1,2,3,6-tétrahydropyridyle, $R_2$ ne représente pas simultanément un atome d'hydrogène,

et

– quand $R_1$ est un radical $y_3$, $R_3$ représente un atome d'hydrogène, A une double liaison et B un radical pipéridyle, $R_2$ ne représente pas simultanément un radical méthyle,

**leurs stéréoisomères possibles,**

**et leurs sels d'addition** à un acide minéral ou organique pharmaceutiquement acceptable, caractérisé en ce que :

l'on fait réagir un composé de formule II :

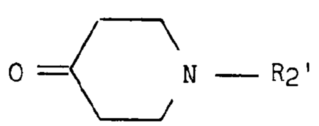

(II)

dans laquelle $R_{2'}$ représente un radical alkyle de 1 à 6 atomes de carbone, ou un radical benzyle, avec un composé de formule III :

$$R_1—X \quad \text{(III)}$$

dans laquelle $R_1$ a la même signification que pour la formule I et X représente un atome de brome, de lithium ou un groupement MgBr, selon la revendication 1, pour former les composés de formule IV :

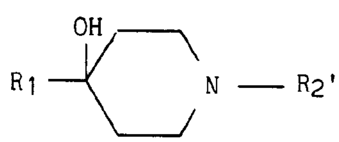

(IV)

dans laquelle $R_1$ a la signification indiquée pour la formule I, selon la revendication 1, et $R_{2'}$ représente un radical alkyle de 1 à 6 atomes de carbone ou un radical benzyle,

que l'on soumet à l'action de l'acide bromhydrique pour former les composés de formule $I_a$ :

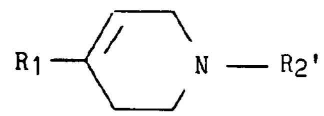

($I_a$)

dans laquelle $R_1$ a la même signification que pour la formule I, selon la revendication 1, et $R_{2'}$ représente un radical alkyle de 1 à 6 atomes de carbone ou un radical benzyle,

et ensuite

- soit

les composés de formule $I_a$ sont soumis à l'action du chloroformiate d'éthyle en présence d'un sel minéral alcalin pour former les composés de formule V :

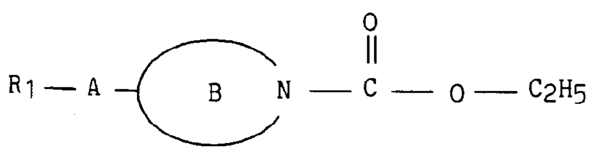

(V)

dans laquelle $R_1$ a la même signification que pour la formule I, selon la revendication 1, A est une simple liaison et B représente un radical 1,2,3,6-tétrahydropyridyle,

- soit

les composés de formule $I_a$ sont d'abord soumis à une hydrogénation catalytique pour former les composés de formule $I_b$ :

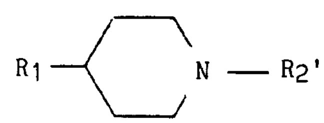

($I_b$)

dans laquelle $R_1$ a la même signification que pour la formule I, selon la revendication 1, et $R_{2'}$ représente un radical alkyle de 1 à 6 atomes de carbone ou un radical benzyle,

puis, à l'action du chloroformiate d'éthyle en présence d'un sel minéral alcalin pour former les composés de formule V,

dans laquelle $R_1$ a la même signification que pour la formule I, selon la revendication 1, et B représente un radical pipéridyle,

dérivés de formule ($I_a$) et ($I_b$) que l'on sépare en leurs stéréoisomères possibles, et/ou que l'on salifie avec un acide organique ou minéral, pharmaceutiquement acceptable, pour former les sels d'addition correspondants.

**2.** Procédé de préparation de composés de formule générale I, selon la revendication 1, caractérisé en ce que l'on fait réagir un composé de formule VI :

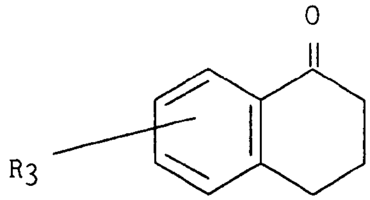

(VI)

dans laquelle $R_3$ a la même signification que pour la formule I, selon la revendication 1,

<u>ou</u>

avec un composé de formule VII,

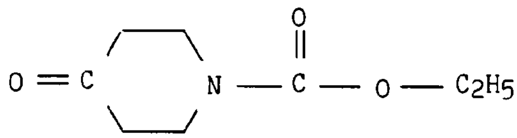

(VII)

pour former les composés de formule V :

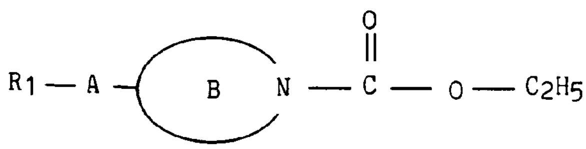

(V)

dans laquelle $R_1$ représente un radical tétrahydronaphtyle de formule $y_3$, A est une double liaison et B représente un radical pipéridyle,

<u>ou</u>

avec un composé de formule VIII :

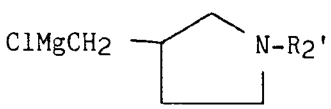

(VIII)

dans laquelle $R_{2'}$ a la même signification que pour la formule II, pour former les composés de formule IX :

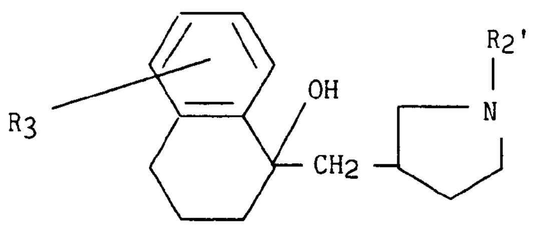

(IX)

dans laquelle $R_3$ à la même signification que pour la formule I, selon la revendication 1, et $R_{2'}$ représente un radical alkyle de 1 à 6 atomes de carbone ou un radical benzyle,

que l'on soumet à l'action de l'acide para-toluènesulfonique pour former les composés de formule $I_c$ et $I_d$ ;

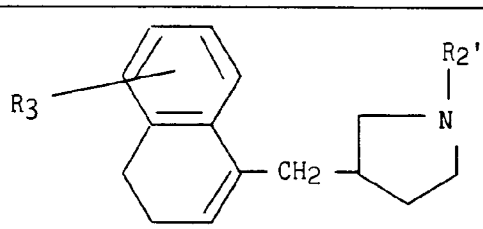

($I_c$)

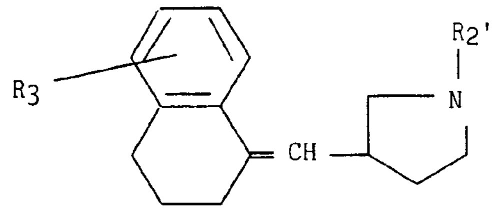

(Id)

dans lesquelles $R_3$ a la même signification que pour la formule I, selon la revendication 1, et la signification de $R_{2'}$ est identique à celle donnée pour la formule II,
lesquels ensuite sont séparés

puis soumis à l'action du chloroformiate d'éthyle en présence d'un sel minéral alcalin pour former les composés de formule V, dans laquelle $R_1$ représente un radical dihydronaphtyle de formule $y_2$, selon la revendication 1, ou un radical tétrahydronaphtyle de formule $y_3$, selon la revendication 1, A est respectivement un radical méthylène ou un radical de formule $z_2$ et B représente un radical pyrrolidinyle,
<u>ou</u>

avec un composé de formule X :

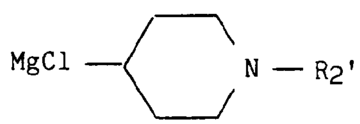

(X)

dans laquelle $R_{2'}$ a la même signification que pour la formule II,
pour former les composés de formule XI :

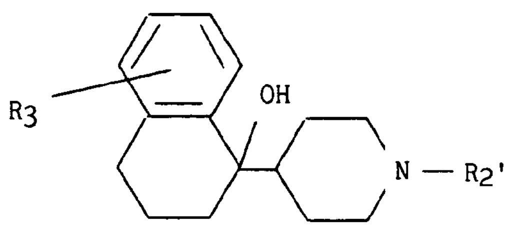

(XI)

dans laquelle la signification de $R_3$ reste identique à celle donnée pour la formule I, selon la revendication 1, et $R_{2'}$ représente un radical benzyle ou un radical alkyle de 1 à 6 atomes de carbone,

lesquels ensuite sont soumis à l'action de l'acide paratoluènesulfonique pour former les composés de formule $I_e$ :

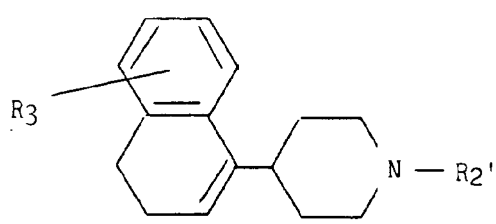

($I_e$)

dans laquelle $R_3$ a la même signification que pour la formule I, selon la revendication 1, et $R_{2'}$ représente un radical alkyle de 1 à 6 atomes de carbone ou un radical benzyle,
lesquels ensuite

- soit

l'on fait réagir avec le chloroformiate d'éthyle pour former les composés de formule V dans laquelle

$R_1$ représente un radical dihydronaphtyle de formule $y_2$ selon la revendication 1, A est une simple liaison et B représente un radical pipéridyle,

- soit

l'on soumet à l'action de tétrachloro 1,2-benzoquinone, pour former les composés de formule $I_f$ :

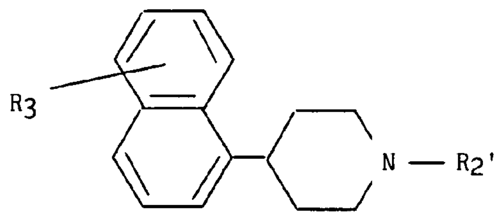

($I_f$)

dans laquelle $R_3$ a la même signification que pour la formule I, selon la revendication 1, et $R_{2'}$ représente un radical alkyle de 1 à 6 atomes de carbone ou un radical benzyle,

lesquels ensuite

l'on fait réagir avec le chloroformiate d'éthyle pour former les composés de formule V dans laquelle $R_1$ représente un radical naphtyle de formule $y_1$, A est une simple liaison et B représente un radical pipéridyle,

et ensuite

l'on soumet les composés de formule V, soit à l'action de l'acide bromhydrique, soit à l'action de l'hydroxyde de potassium, pour former les composés de formule $I_g$ :

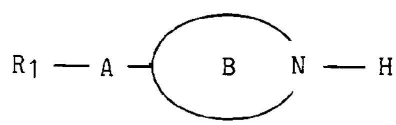

($I_g$)

dans laquelle la signification de $R_1$, A et B reste identique à celle donnée pour la formule I, selon la revendication 1,

lesquels ensuite l'on fait réagir avec un composé de formule XII :

$$Br\text{-}(CH_2)_{n-1}\text{-}CN \quad (XII)$$

dans laquelle n a la même signification que pour la formule I, selon la revendication 1, pour former les composés de formule $I_h$ :

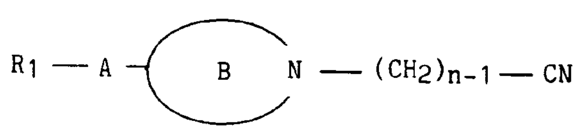

($I_h$)

dans laquelle $R_1$, A, B et n ont la même signification que pour la formule I, selon la revendication 1,

lesquels ensuite sont soumis à l'action d'hydrure de lithium et d'aluminium pour former les composés de formule $I_i$ :

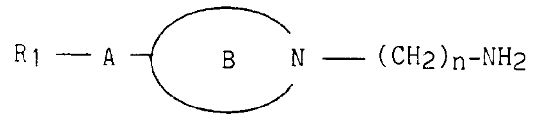 (Ii)

dans laquelle la signification de $R_1$, A, B et n reste identique à celle donnée pour la formule I, selon la revendication 1,
que l'on fait réagir avec un composé de formule XIII :

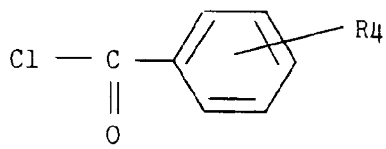 (XIII)

dans laquelle la signification de $R_4$ reste identique à celle donnée pour la formule I, selon la revendication 1,
pour former les composés de formule $I_j$ :

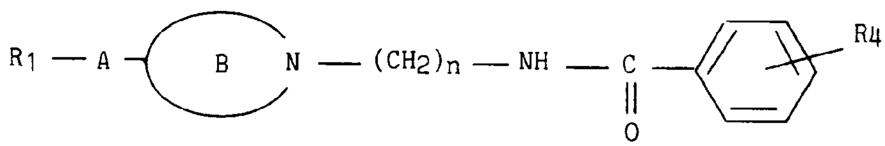 (Ij)

dans laquelle $R_1$, A, B, n et $R_4$ ont la même signification que pour la formule I, selon la revendication 1,

et ensuite

l'on sépare les composés de formule $I_c$-$I_j$ en leurs stéréoisomères possibles,
et/ou l'on salifie avec un acide organique ou minéral, pharmaceutiquement acceptable, pour former les sels d'addition correspondants.

3. Procédé de préparation selon la revendication 1 de la 1-[2-(4-fluorobenzamido) éth-1-yl] 4-(7-méthoxy-napht-1-yl) pipéridine, ainsi que ses sels d'addition à un acide minéral ou organique, pharmaceutiquement acceptable.

**Revendications pour l'état contractant suivant: GR**

1. Procédé de préparation des composés de formule I :

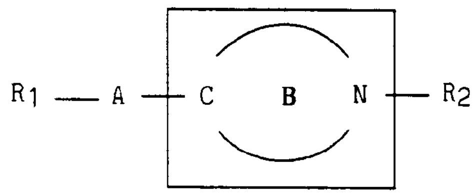

(I)

dans laquelle

- $R_1$ représente un radical naphtyle, de formule $y_1$ :

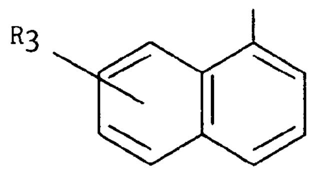

($y_1$)

un radical dihydronaphtyle, de formule $y_2$ :

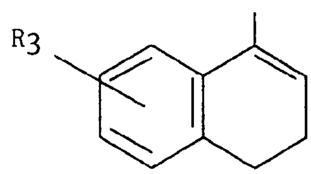

($y_2$)

un radical tétrahydronaphtyle de formule $y_3$ :

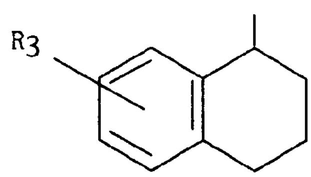

($y_3$)

(dans lesquelles $R_3$ représente un atome d'hydrogène, un atome d'halogène, un radical alkyle de 1 à 6 atomes de carbone, un radical hydroxy ou un radical alcoxy de 1 à 6 atomes de carbone), un radical quinol-3-yle (éventuellement substitué sur le cycle benzénique par un ou plusieurs atomes d'halogène, radicaux alkyle de 1 à 6 atomes de carbone, radicaux hydroxy, ou radicaux alcoxy de 1 à 6 atomes de carbone), ou un radical 1,4-benzodioxan-5-yl,

- A représente une simple liaison, une double liaison, (à condition toutefois que $R_1$ représente un radical tétrahydronaphtyle), un radical méthylène, un radical de formule $z_1$ :

$$-CH= \qquad (z_1)$$

ou un radical de formule $z_2$ :

$$=CH- \qquad (z_2)$$

(à condition toutefois que dans ce cas, $R_1$ représente un radical tétrahydronaphtyle),

- le cycle B représente un radical pipéridyle (à condition toutefois que dans ce cas A représente une simple ou une double liaison), un radical pyrrolidinyle (à condition toutefois que dans ce cas A représente un radical méthylène, un radical $z_1$ ou un radical $z_2$), ou un radical 1,2,3,6-tétrahydropyridyle (à condition toutefois que dans ce cas A représente une simple liaison),

$R_2$ représente :

– un atome d'hydrogène, un radical benzyle, un radical alkyle de 1 à 6 atomes de carbone, à condition que dans l'un de ces cas $R_1$ soit différent d'un radical naphtyle ($y_1$), dihydronaphtyle ($y_2$) ou tétrahydro-

naphtyle ($y_3$) et B soit différent d'un radical pipéridinyl,
– un radical aminoalkyle de 1 à 6 atomes de carbone, un radical cyanoalkyle de 1 à 6 atomes de carbone ou un radical de formule $w_1$ :

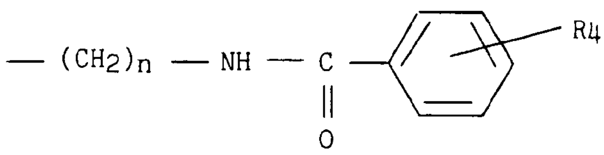

($w_1$)

(dans laquelle :
n est égal à 1-6
et
$R_4$ représente un atome d'hydrogène, un atome d'halogène, un radical alkyle de 1 à 6 atomes de carbone ou un radical alcoxy de 1 à 6 atomes de carbone),
à condition toutefois que
– quand $R_1$ est un radical $y_2$, $R_3$ représente un atome d'hydrogène, A une simple liaison et B un radical pipéridyle, $R_2$ ne représente pas simultanément un radical méthyle,
et
– quand $R_2$ est un radical $y_1$, $R_3$ représente un atome d'hydrogène, A une simple liaison et B un radical 1,2,3,6-tétrahydropyridyle, $R_2$ ne représente pas simultanément un atome d'hydrogène,
et
– quand $R_1$ est un radical $y_3$, $R_3$ représente un atome d'hydrogène, A une double liaison et B un radical pipéridyle, $R_2$ ne représente pas simultanément un radical méthyle,
**leurs stéréoisomères possibles,**
**et leurs sels d'addition** à un acide minéral ou organique pharmaceutiquement acceptable, caractérisé en ce que :
l'on fait réagir un composé de formule II :

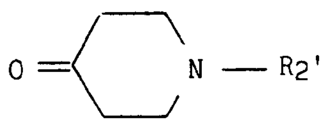

(II)

dans laquelle $R_{2'}$ représente un radical alkyle de 1 à 6 atomes de carbone, ou un radical benzyle, avec un composé de formule III :

$$R_1—X \quad (III)$$

dans laquelle $R_1$ a la même signification que pour la formule I et X représente un atome de brome, de lithium ou un groupement MgBr, selon la revendication 1, pour former les composés de formule IV :

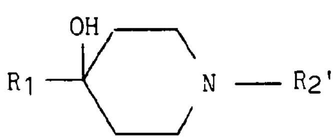

(IV)

dans laquelle $R_1$ a la signification indiquée pour la formule I, selon la revendication 1, et $R_{2'}$ représente un radical alkyle de 1 à 6 atomes de carbone ou un radical benzyle,
que l'on soumet à l'action de l'acide bromhydrique pour former les composés de formule $I_a$ :

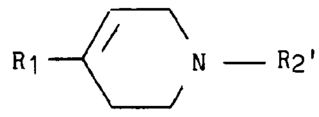

(Ia)

dans laquelle $R_1$ a la même signification que pour la formule I, selon la revendication 1, et $R_{2'}$ représente un radical alkyle de 1 à 6 atomes de carbone ou un radical benzyle,

et ensuite

- soit

les composés de formule $I_a$ sont soumis à l'action du chloroformiate d'éthyle en présence d'un sel minéral alcalin pour former les composés de formule V :

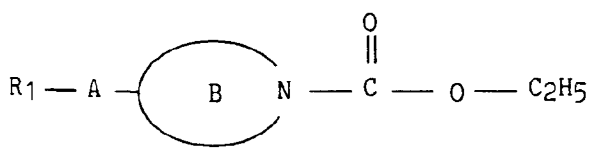

(V)

dans laquelle $R_1$ a la même signification que pour la formule I, selon la revendication 1, A est une simple liaison et B représente un radical 1,2,3,6-tétrahydropyridyle,

- soit

les composés de formule $I_a$ sont d'abord soumis à une hydrogénation catalytique pour former les composés de formule $I_b$ :

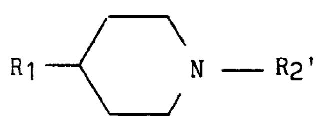

(Ib)

dans laquelle $R_1$ a la même signification que pour la formule I, selon la revendication 1, et $R_{2'}$ représente un radical alkyle de 1 à 6 atomes de carbone ou un radical benzyle,

puis, à l'action du chloroformiate d'éthyle en présence d'un sel minéral alcalin pour former les composés de formule V,

dans laquelle $R_1$ a la même signification que pour la formule I, selon la revendication 1, et B représente un radical pipéridyle,

dérivés de formule (Ia) et (Ib) que l'on sépare en leurs stéréoisomères possibles, et/ou que l'on salifie avec un acide organique ou minéral, pharmaceutiquement acceptable, pour former les sels d'addition correspondants.

2. Procédé de préparation de composés de formule générale I, selon la revendication 1, caractérisé en ce que l'on fait réagir un composé de formule VI :

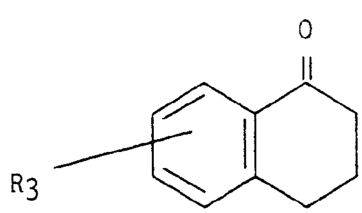

(VI)

dans laquelle $R_3$ a la même signification que pour la formule I, selon la revendication 1,

ou

avec un composé de formule VII,

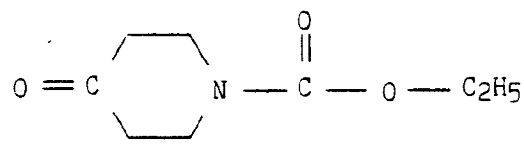

(VII)

pour former les composés de formule V :

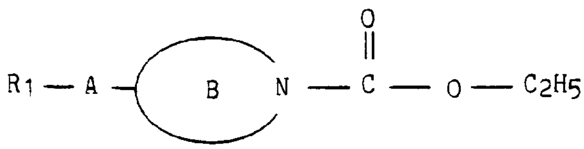

(V)

dans laquelle $R_1$ représente un radical tétrahydronaphtyle de formule $y_3$, A est une double liaison et B représente un radical pipéridyle,

ou

avec un composé de formule VIII :

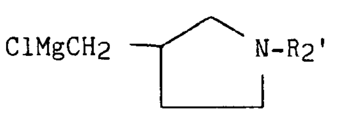

(VIII)

dans laquelle $R_{2'}$ a la même signification que pour la formule II, pour former les composés de formule IX :

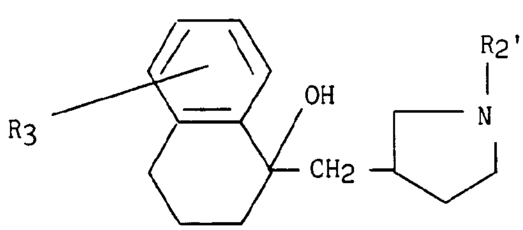

(IX)

dans laquelle $R_3$ à la même signification que pour la formule I, selon la revendication 1, et $R_{2'}$ représente un radical alkyle de 1 à 6 atomes de carbone ou un radical benzyle,

que l'on soumet à l'action de l'acide para-toluènesulfonique pour former les composés de formule $I_c$ et $I_d$ :

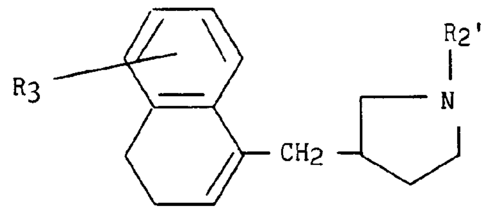

(Ic)

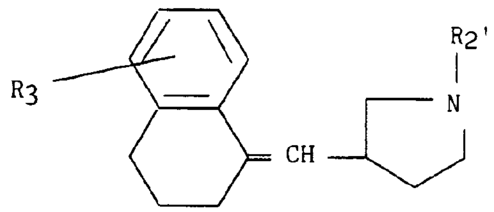

(Id)

dans lesquelles $R_3$ a la même signification que pour la formule I, selon la revendication 1, et la signification de $R_{2'}$ est identique à celle donnée pour la formule II,
lesquels ensuite sont séparés

puis soumis à l'action du chloroformiate d'éthyle en présence d'un sel minéral alcalin pour former les composés de formule V, dans laquelle $R_1$ représente un radical dihydronaphtyle de formule $y_2$, selon la revendication 1, ou un radical tétrahydronaphtyle de formule $y_3$, selon la revendication 1, A est respectivement un radical méthylène ou un radical de formule $z_2$ et B représente un radical pyrrolidinyle,
<u>ou</u>

avec un composé de formule X :

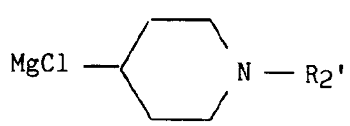

(X)

dans laquelle $R_{2'}$ a la même signification que pour la formule II,
pour former les composés de formule XI :

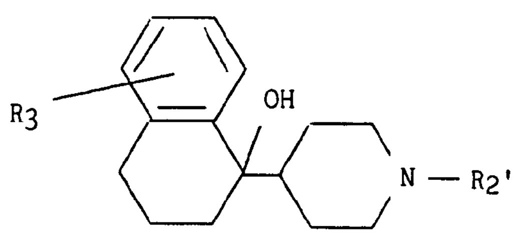

(XI)

dans laquelle la signification de $R_3$ reste identique à celle donnée pour la formule I, selon la revendication 1, et $R_{2'}$ représente un radical benzyle ou un radical alkyle de 1 à 6 atomes de carbone,

lesquels ensuite sont soumis à l'action de l'acide paratoluènesulfonique pour former les composés de formule $I_e$ :

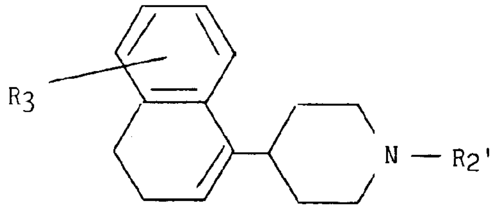

($I_e$)

dans laquelle $R_3$ a la même signification que pour la formule I, selon la revendication 1, et $R_{2'}$ représente un radical alkyle de 1 à 6 atomes de carbone ou un radical benzyle,
lesquels ensuite

- soit

l'on fait réagir avec le chloroformiate d'éthyle pour former les composés de formule V dans laquelle $R_1$ représente un radical dihydronaphtyle de formule $y_2$ selon la revendication 1, A est une simple liaison et B représente un radical pipéridyle,

- soit

l'on soumet à l'action de tétrachloro 1,2-benzoquinone, pour former les composés de formule $I_f$ :

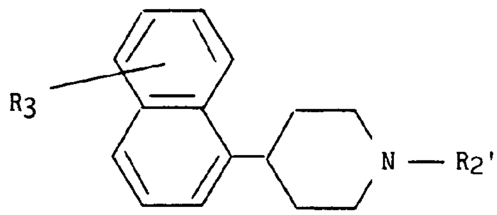

($I_f$)

dans laquelle $R_3$ a la même signification que pour la formule I, selon la revendication 1, et $R_{2'}$ représente un radical alkyle de 1 à 6 atomes de carbone ou un radical benzyle,

lesquels ensuite

l'on fait réagir avec le chloroformiate d'éthyle pour former les composés de formule V dans laquelle $R_1$ représente un radical naphtyle de formule $y_1$, A est une simple liaison et B représente un radical pipéridyle,

et ensuite

l'on soumet les composés de formule V, soit à l'action de l'acide bromhydrique, soit à l'action de l'hydroxyde de potassium, pour former les composés de formule $I_g$ :

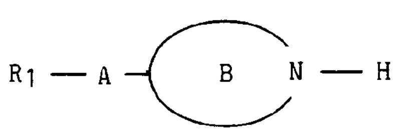

($I_g$)

dans laquelle la signification de $R_1$, A et B reste identique à celle donnée pour la formule I, selon la revendication 1,

lesquels ensuite l'on fait réagir avec un composé de formule XII :

$$Br\text{-}(CH_2)_{n-1}\text{-}CN \quad (XII)$$

dans laquelle n a la même signification que pour la formule I, selon la revendication 1, pour former

les composés de formule $I_h$ :

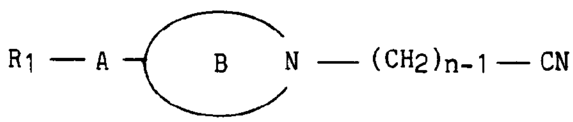

$(I_h)$

dans laquelle $R_1$, A, B et n ont la même signification que pour la formule I, selon la revendication 1,

lesquels ensuite sont soumis à l'action d'hydrure de lithium et d'aluminium pour former les composés de formule $I_i$ :

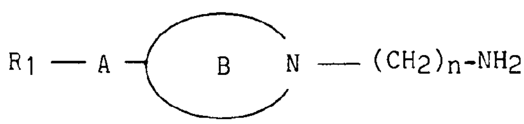

$(I_i)$

dans laquelle la signification de $R_1$, A, B et n reste identique à celle donnée pour la formule I, selon la revendication 1,
que l'on fait réagir avec un composé de formule XIII :

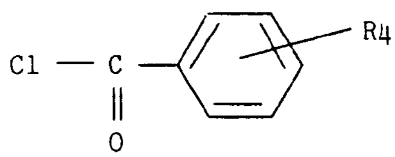

(XIII)

dans laquelle la signification de $R_4$ reste identique à celle donnée pour la formule I, selon la revendication 1,
pour former les composés de formule $I_j$ :

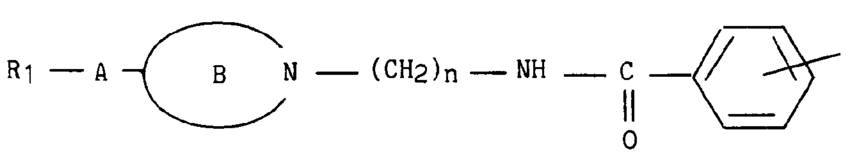

$(I_j)$

dans laquelle $R_1$, A, B, n et $R_4$ ont la même signification que pour la formule I, selon la revendication 1,

et ensuite

l'on sépare les composés de formule $I_c$-$I_j$ en leurs stéréoisomères possibles,

et/ou l'on salifie avec un acide organique ou minéral, pharmaceutiquement acceptable, pour former les sels d'addition correspondants.

3. Procédé de préparation selon la revendication 1 de la 1-[2-(4-fluorobenzamido) éth-1-yl] 4-(7-méthoxy-napht-1-yl) pipéridine, ainsi que ses sels d'addition à un acide minéral ou organique, pharmaceutiquement acceptable.

FIGURE 1
Composé de l'exemple 18
30 µg/kg i.v.

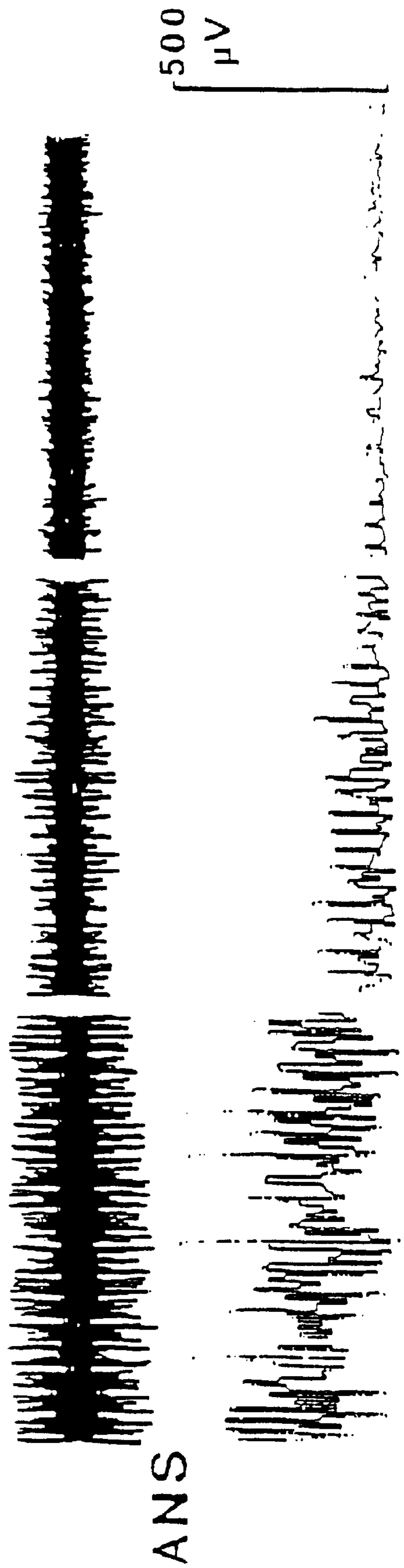

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande: EP 91 40 1915

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 69, 1968, page 273, résumé no. 2835z, Columbus, Ohio, US; A. RACZKA: "Preparation of cycloalkyl and benzocycloalkyl derivatives of N,N-dimethylpropylamine and N-methylpiperidine", & ACTA POL. PHARM. 1967, 24(5), 489-96<br>* Composés de formule VII,X * | 1 | C 07 D 211/22<br>C 07 D 211/14<br>C 07 D 211/70<br>C 07 D 211/12<br>C 07 D 207/20<br>C 07 D 401/04<br>C 07 D 405/04<br>C 07 D 207/08<br>A 61 K 31/445 |
| X | GB-A-1 110 087 (ABBOTT)<br>* Revendication 5 * | 1 | |
| X | GB-A-2 041 918 (RICHARDSON-MERRELL)<br>* En entier; voir revendications 13,16 * | 1 | |
| X | US-A-2 498 431 (LEE et al.)<br>* En entier; revendication 1 * | 1 | |
| X | EP-A-0 229 391 (EISAI)<br>* Revendications 1,25 *<br>-/- | 1 | |
| | | | **DOMAINES TECHNIQUES RECHERCHES (Int. Cl.5)**<br>C 07 D 207/00<br>C 07 D 211/00<br>C 07 D 401/00<br>C 07 D 405/00 |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches compl-tes: 2

Revendications ayant fait l'objet de recherches incompl-tes: 1,3-8

Revendications n'ayant pas fait l'objet de recherches:

Raison pour la limitation de la recherche:

Obscurités:

La formule (I) de la revendication 1 n'est pas claire et concise (Art. 84), et par la cascade des nombreux 'disclaimers' divient difficile à comprendre ainsi seulement des derivés de la pyrrolidine-3 et de la piperidine-4 (ou de la 1,2,3,6-tetrahydro-pyridine) ont fait l'objet de la recherche.

| Lieu de la recherche | Date d'achèvement de la recherche | Examinateur |
|---|---|---|
| LA HAYE | 02-09-1991 | KISSLER B.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0408)

Page 2

Office européen des brevets

RAPPORT PARTIEL DE RECHERCHE EUROPEENNE

Numero de la demande

EP 91 40 1915

| Catégorie | Citation du document avec indication, en cas de besoin, des parties pertinentes | Revendication concernée | CLASSEMENT DE LA DEMANDE (Int. Cl. 5) |
|---|---|---|---|
| X | EP-A-0 296 560 (EISAI)<br>* Revendication 1 * | 1 | |
| X | EP-A-0 042 781 (PHARMIDUSTRIE)<br>* Revendication 1 * | 1 | |
| X | GB-A-2 051 799 (SANDOZ)<br>* En entier; voir page 5, lignes 20-25; revendications 1,4-6 * | 1 | |
| X | GB-A-2 185 980 (SANDOZ)<br>* Revendication 1; exemple 9 * | 1 | |
| A | US-A-4 757 079 (GRAY)<br>* En entier * | 1,5-8 | DOMAINES TECHNIQUES RECHERCHES (Int.Cl. ) |
| A | EP-A-0 372 776 (PFIZER)<br>* En entier * | 1 | |
| X | EP-A-0 398 413 (DUPHAR)<br>* En entier; voir composés 4-7,19,48,49 * | 1-2 | |
| X | EP-A-0 325 963 (ABBOTT)<br>* En entier; voir exemples 94,110 * | 1 | |
| A | J. CHEM. SOC. C., vol. 69, no. 2, 1969, pages 217-222; P.J. HATTERSLEY et al.: "Some alkylation and grignard reactions with 1-tetralones and related compounds"<br>* En entier; voir tableaux 1,2 * | 1 | |
| X | J. MED. CHEM. vol. 33, 1990, pages 3133-3138, American Chemical Society; S.M.N. EFANGE et al.: "Flexible N-methyl-4-phenyl-1,2,3,6-tetrahydropyridine analogues: Synthesis and monoamine oxidase catalyzed bioactivation"<br>* Formule 3i * | 1 | |
| X | DE-A-2 834 372 (SQUIBB)<br>* Exemple 110B, page 74 * | 1 | |

EPO FORM 1503 03.82 (P0411)